# FASCICULE DE BREVET EUROPEEN

(11) **EP 2 257 619 B1**
(45) Date de publication et mention de la délivrance du brevet: **16.12.2015**
(21) Numéro de dépôt: 09721138.7
(22) Date de dépôt: 13.03.2009
(51) Int. Cl.: C12N 1/19, C12N 1/18, C12R 1/865

(54) **UTILISATION D'UN SUBSTITUT CARBONE POUR LA PRODUCTION DE LEVURES**
VERWENDUNG EINES KOHLENSTOFFHALTIGEN SUBSTITUTS FÜR DIE HERSTELLUNG VON HEFE
USE OF A CARBONACEOUS SUBSTITUTE FOR THE PRODUCTION OF YEAST

(30) Priorité: 14.03.2008 FR 0801398
(43) Date de publication de la demande: 08.12.2010
(73) Titulaire: Lesaffre et Compagnie, 75001 Paris (FR)
(72) Inventeur: FUENTES, Jean-Luc, New Berlin-WI 53151 (US); PARASIE, Georges, René, Marcel, F-59320 Sequedin (FR); POILPRE, Emmanuel, F-22600 Loudeac (FR); QUIPOURT-ISNARD, Anne-Dominique, F-59700 Marcq-en-Baroeul (FR); STIEN, Gilles, Georges, Albert, F-59830 Bourghelles (FR)
(74) Mandataire: Cabinet Plasseraud
(86) Numéro de dépôt international: PCT/IB2009/000511
(87) Numéro de publication internationale: WO 2009/112940

(56) Documents cités:
- WO-A-2008/006037
- FERREIRA ET AL: "Glucose repression over Saccharomyces cerevisiae glycerol/H<+> symporter gene STL1 is overcome by high temperature" FEBS LETTERS, ELSEVIER, AMSTERDAM, NL, vol. 581, no. 9, 21 avril 2007 (2007-04-21), pages 1923-1927, XP022041902 ISSN: 0014-5793
- YAAKOV GILAD ET AL: "Combination of two activating mutations in one HOG1 gene forms hyperactive enzymes that induce growth arrest." MOLECULAR AND CELLULAR BIOLOGY, vol. 23, no. 14, juillet 2003 (2003-07), pages 4826-4840, XP002503424 ISSN: 0270-7306
- LAGES F ET AL: "Contribution to the physiological characterization of glycerol active uptake in Saccharomyces cerevisiae" BIOCHIMICA ET BIOPHYSICA ACTA. BIOENERGETICS, AMSTERDAM, NL, vol. 1322, no. 1, 10 novembre 1997 (1997-11-10), pages 8-18, XP004338607 ISSN: 0005-2728
- OLIVEIRA R ET AL: "Fps1p channel is the mediator of the major part of glycerol passive diffusion in Saccharomyces cerevisiae: artefacts and re-definitions" BIOCHIMICA ET BIOPHYSICA ACTA. BIOMEMBRANES, AMSTERDAM, NL, vol. 1613, no. 1-2, 27 juin 2003 (2003-06-27), pages 57-71, XP004433592 ISSN: 0005-2736
- DOMBEK KENNETH M ET AL: "Cyclic AMP-dependent protein kinase inhibits ADH2 expression in part by a decreasing expression of the transcription factor gene ADR1" MOLECULAR AND CELLULAR BIOLOGY, vol. 17, no. 3, 1997, pages 1450-1458, XP002503425 ISSN: 0270-7306
- FERREIRA CELIA ET AL: "A member of the sugar transporter family, Stl1p is the glycerol/H+ symporter in Saccharomyces cerevisiae" MOLECULAR BIOLOGY OF THE CELL, vol. 16, no. 4, avril 2005 (2005-04), pages 2068-2076, XP002503426 ISSN: 1059-1524

## Description

### DOMAINE TECHNIQUE

La présente invention concerne la production de levures, notamment à l'échelle industrielle. La présente invention a pour objet l'utilisation d'un substrat carboné permettant de remplacer partiellement ou totalement l'utilisation de sucre pour la production de levures, notamment à l'échelle industrielle.

### ARRIERE-PLAN TECHNOLOGIQUE

La production de levures suppose l'apport de différents éléments nutritifs capables d'assurer leur croissance. Ces différents éléments nutritifs sont en particulier le carbone, l'azote, le phosphore, le soufre, des minéraux et des vitamines. Parmi les milieux naturels pouvant être utilisés pour la production de levure, les mélasses occupent une place de choix. En effet, les mélasses constituent un milieu relativement complet capable d'assurer l'apport des ces éléments nutritifs. La composition des mélasses est en moyenne la suivante : de 66 % à 73 % de sucres, de 15 % à 23 % de composés organiques et de 10 % à 12 % de composés minéraux (en pourcentages de matières sèches totales). La source carbonée fournie par les mélasses est essentiellement constituée de saccharose ou des sucres résultant de son hydrolyse (glucose et fructose).

Cependant, la raréfaction des mélasses de canne et de betterave, en particulier liée à leur utilisation massive pour la production de bioéthanol, met en péril ce type de production. Il est en particulier nécessaire de trouver de nouveaux substrats carbonés en substitution du saccharose fourni par les mélasses.

La production de levures peut également être effectuée sur des sirops de glucose ou de fructose, par exemple issus de l'hydrolyse de l'amidon et provenant notamment de céréales (maïs, blé, riz) ou de la pomme de terre. Cependant, ces sirops sont d'un coût relativement élevé et ne représentent pas une solution économiquement rentable pour remplacer le saccharose des mélasses dans le cadre d'une production industrielle de levure.

La levure du genre *Saccharomyces* est capable de synthétiser du glycérol en anaérobiose ou en situation de stress prononcé, par exemple en cas de stress osmotique ou thermique. La levure *Saccharomyces* est par ailleurs capable de dégrader le glycérol, mais dans un métabolisme purement oxydatif et en l'absence de répression catabolique par des sucres. Ainsi, la levure *Saccharomyces,* mise en présence de glycérol et de sucre dans une culture batch, n'est pas capable de consommer le glycérol, tant qu'il reste du sucre dans le milieu.

Par ailleurs, dans un métabolisme oxydatif, la levure *Saccharomyces* cultivée sur un substrat carboné constitué uniquement de glycérol consomme le glycérol extrêmement lentement, ce qui n'est pas compatible avec une production de levure à l'échelle industrielle. (Lages et Lucas, 1997, Biochimica et Biophysica Acta).

Ferreira et al. (FEBS Letters, 2007, 581: 1923-1927) s'intéressent à la dépendance de l'activité de la protéine Stllp chez la levure vis-à-vis de la température, et décrit une souche de *Saccharomyces* qui exprime le gène STL1 en présence de glucose mais qui n'a pas de répression de glucose à une température élevée. Cette souche de levure ne consomme pas de glycérol, mais à 37°C, réagit à un stress thermique en accumulant du glycérol.

Yaakov Gilad et al. (Mol. Cell. Biol., 2003, 23: 4826-4840) ont publié une étude sur le rôle de la protéine kinase Hog1 de *S. cerevisiae*. Ils décrivent un mutant Hog1 (D170A, F318S) qui induit l'expression du gène STL1. Cependant, la croissance du mutant est inhibée, ce qui exclut toute consommation de glycérol.

Enfin, Lages et al. (Biochimica et Biophysica Acta, 1997, 1322: 8-18) ont montré que lorsqu'un "starter" contenant 0,2% de glucose est ajouté à un milieu de culture contenant du glycérol, *S. cerevisiae* consomme le glucose avant de consommer le glycérol.

Il y a donc un réel besoin de nouvelles souches pour la production de levures qui permettent de remplacer totalement ou partiellement le sucre en tant que substrat carboné, avec des rendements de production de levure compatibles avec une exploitation économique et industrielle, et/ou sans perte de qualité des levures produites.

### RESUME DE L'INVENTION

La présente invention a pour objet de fournir de nouvelles souches de *Saccharomyces* capables d'être produites sur un substrat carboné permettant de remplacer totalement et/ou partiellement l'utilisation de sucre.

Un objet de l'invention est également l'utilisation de ces souches pour la production de levures, notamment à l'échelle industrielle.

Un autre objet de l'invention concerne un procédé de production de levures du genre *Saccharomyces* sur un substrat carboné permettant de remplacer totalement ou partiellement l'utilisation de sucre, ledit procédé permettant également d'obtenir un rendement similaire à celui d'une production sur un substrat de type sucre et/ou permettant d'obtenir des levures de qualité semblable.

La présente invention a pour objet une souche de *Saccharomyces*, caractérisée en ce qu'en présence d'un milieu de culture comprenant un mélange de glycérol et de sucre, en condition aérobie, elle consomme au moins 80 % du glycérol dudit mélange tout en consommant du sucre simultanément, ledit mélange comprenant une proportion de glycérol d'au moins 20 % en équivalent saccharose, la somme des proportions de glycérol et de sucre dudit mélange étant égale à 100 % en équivalent saccharose, la souche de Saccharomyces étant une souche modifiée génétiquement pour sur-exprimer le gène STL1 en présence de sucre ou étant choisie parmi la souche déposée le 3 mars 2009 auprès de la CNCM sous le numéro CNCM I-4129, la souche déposée le 3 mars 2009 auprès de la CNCM sous le numéro CNCM I-4130 et la souche déposée le 3 mars 2009 auprès de la CNCM sous le numéro CNCM I-4131.

La présente invention a également pour objet une souche telle que définie ci-dessus, caractérisée en ce qu'elle consomme au moins 85 % du glycérol du mélange, de préférence au moins 90 % du glycérol du mélange, plus préférentiellement au moins 95 % du glycérol du mélange, encore plus préférentiellement au moins 98 % du glycérol du mélange.

La présente invention a également pour objet une souche telle que définie ci-dessus, caractérisée en ce que ledit mélange comprend une proportion de glycérol d'au moins 30 % en équivalent saccharose, de préférence au moins 40 % en équivalent saccharose, plus préférentiellement au moins 50 % en équivalent saccharose, encore plus préférentiellement au moins 60 % en équivalent saccharose, encore plus préférentiellement au moins 70 % en équivalent saccharose, encore plus préférentiellement au moins 80 % en équivalent saccharose encore plus préférentiellement au moins 90 % en équivalent saccharose.

La présente invention concerne en particulier une souche telle que définie ci-dessus, caractérisée en ce qu'elle est choisie parmi les espèces *cerevisiae*, *boulardii*, *carlsbergensis* et *uvarum*.

La présente invention a également pour objet l'utilisation d'une souche telle que définie ci-dessus pour la production de levures en présence d'un mélange de glycérol et de sucre comprenant une proportion de glycérol d'au moins 5 % en équivalent saccharose, la somme des proportions de glycérol et de sucre dudit mélange étant égale à 100 % en équivalent saccharose.

La présente invention a pour objet l'utilisation d'un mélange de glycérol et de sucre pour la production de levures à partir d'au moins une souche de Saccharomyces selon l'invention, ledit mélange comprenant une proportion de glycérol d'au moins 5 % en équivalent saccharose, la somme des proportions de glycérol et de sucre dudit mélange étant égale à 100 % en équivalent saccharose.

La présente invention a également pour objet un procédé de production de levures comprenant les étapes de :
introduction d'une souche de levure du genre *Saccharomyces* selon l'invention dans un fermenteur, et
multiplication de ladite souche en condition aérobie dans un milieu de culture comprenant un mélange de glycérol et de sucre, ledit mélange comprenant une proportion de glycérol d'au moins 5 % en équivalent saccharose, la somme des proportions de glycérol et de sucre dudit mélange étant égale à 100 % en équivalent saccharose.

La présente invention a particulièrement pour objet un procédé tel que défini ci-dessus, caractérisé en ce qu'au moins 80 % du glycérol dudit mélange est consommé par ladite souche simultanément à la consommation de sucre, de préférence au moins 85 % du glycérol dudit mélange, plus préférentiellement au moins 90 % du glycérol dudit mélange, encore plus préférentiellement au moins 95 % du glycérol dudit mélange, encore plus préférentiellement au moins 98 % du glycérol dudit mélange.

La présente invention a pour objet un procédé tel que défini ci-dessus, caractérisé en ce que la multiplication de ladite souche est réalisée selon un mode semi-continu ou selon un mode continu.

La présente invention a également pour objet un procédé tel que défini ci-dessus, caractérisé en ce que ledit mélange comprend une proportion de glycérol d'au moins 8 % en équivalent saccharose, de préférence au moins 10 % en équivalent saccharose, plus préférentiellement au moins 12 % en équivalent saccharose, encore plus préférentiellement au moins 15 % en équivalent saccharose, encore plus préférentiellement au moins 17 % en équivalent saccharose, encore plus préférentiellement au moins 20 % en équivalent saccharose.

La présente invention a particulièrement pour objet un procédé tel que défini ci-dessus, caractérisé en ce que ledit mélange comprend une proportion de glycérol d'au moins 30 % en équivalent saccharose.

La présente invention a pour objet un procédé tel que défini ci-dessus, caractérisé en ce que ledit mélange comprend une proportion de glycérol d'au moins 40 % en équivalent saccharose, de préférence au moins 50 % en équivalent saccharose, plus préférentiellement au moins 60 % en équivalent saccharose, encore plus préférentiellement au moins 70 % en équivalent saccharose, encore plus préférentiellement au moins 80 % en équivalent saccharose, encore plus préférentiellement au moins 90 % en équivalent saccharose.

### BREVE DESCRIPTION DES FIGURES

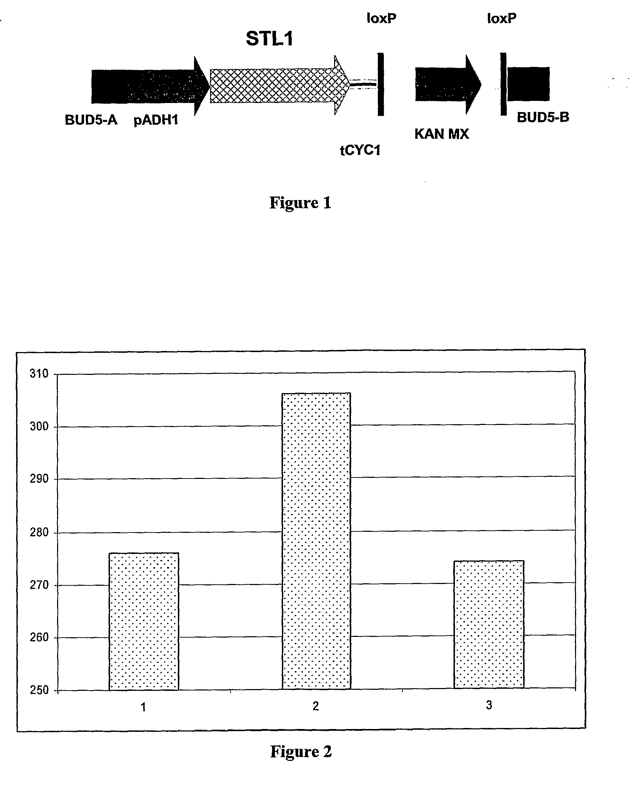

Figure 1 : Schéma de la cassette d'expression utilisée pour intégrer le gène STL1 dans le génome de souches de levures.

Le gène STL1 est sous contrôle du promoteur pADH1 et il est suivi du terminateur CYC1 de levure. Deux fragments recombinogéniques (BUD5-A et BUD5-B) de 500 nucléotides chacun encadrent la cassette d'expression, afin d'assurer l'insertion par recombinaison homologue de la cassette dans le génome de la levure, après transformation, au niveau du locus BUD5 (YCR038c) situé sur le chromosome III. Le gène conférant la résistance à la généticine KANMX est inséré en aval du gène STL1, encadré de deux sites loxP.

Figure 2 : Niveau d'expression du gène STL1 dans les souches génétiquement modifiées pour exprimer le gène STL1, après culture en présence de sucre.

Le graphique représente le rapport du taux d'ARNm de souches de levure mutées exprimant le gène STL1 sur le taux d'ARNm de la souche non mutée correspondante (en ordonnées). 1 : rapport du taux d'ARNm de la souche déposée à la CNCM sous le numéro I-3887 sur celui de la souche non mutée NCYC 996 ; 2 : rapport du taux d'ARNm de la souche déposée à la CNCM sous le numéro I-3888 sur celui de la souche non mutée NCYC 995 ; 3 : rapport du taux d'ARNm de la souche déposée à la CNCM sous le numéro I-3886 sur celui de la souche non mutée I-3399.

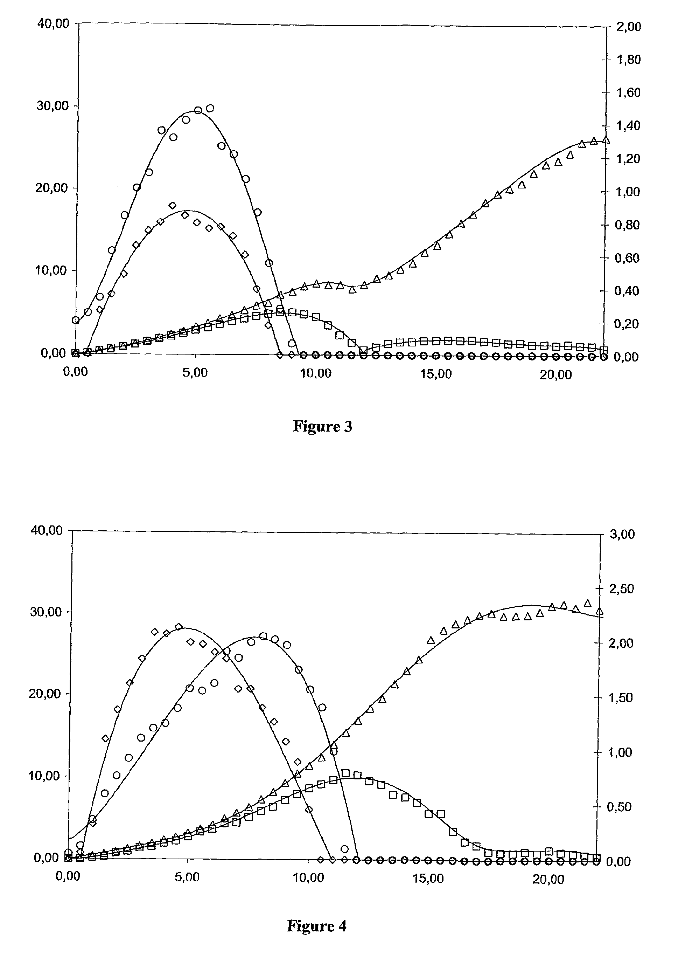

Figure 3 : Quantité résiduelle de glycérol, après culture en mode semi-continu, de la souche I-3887 en comparaison avec celle de la souche non mutée NCYC 996, en fonction du temps. Le mélange comportait 20 % de glycérol en équivalent saccharose. L'axe des ordonnées à gauche indique la quantité de glycérol (en g) et l'axe des ordonnées à droite la quantité d'éthanol (en g) dans le milieu débarrassé des levures. L'axe des abscisses indique le temps (en heure). La courbe en triangle représente la quantité résiduelle de glycérol dans le milieu débarrassé des levures au cours de la culture de la souche non mutée NCYC 996 et la courbe en carré celle de la souche I-3887. La courbe en cercle représente la quantité résiduelle d'éthanol dans le milieu débarrassé des levures au cours de la culture de la souche non mutée NCYC 996 et la courbe en losange celle de la souche I-3887.

Figure 4 : Quantité résiduelle de glycérol, après culture en mode semi-continu, de la souche I-3888 en comparaison avec celle de la souche non mutée NCYC 995, en fonction du temps. Le mélange comportait 20 % de glycérol en équivalent saccharose. L'axe des ordonnées à gauche indique la quantité de glycérol (en g) et l'axe des ordonnées à droite la quantité d'éthanol (en g) dans le milieu débarrassé des levures. L'axe des abscisses indique le temps (en heure). La courbe en triangle représente la quantité résiduelle de glycérol dans le milieu débarrassé des levures au cours de la culture de la souche non mutée NCYC 995 et la courbe en carré celle de la souche I-3888. La courbe en cercle représente la quantité résiduelle d'éthanol dans le milieu débarrassé des levures au cours de la culture de la souche non mutée NCYC 995 et la courbe en losange celle de la souche I-3888.

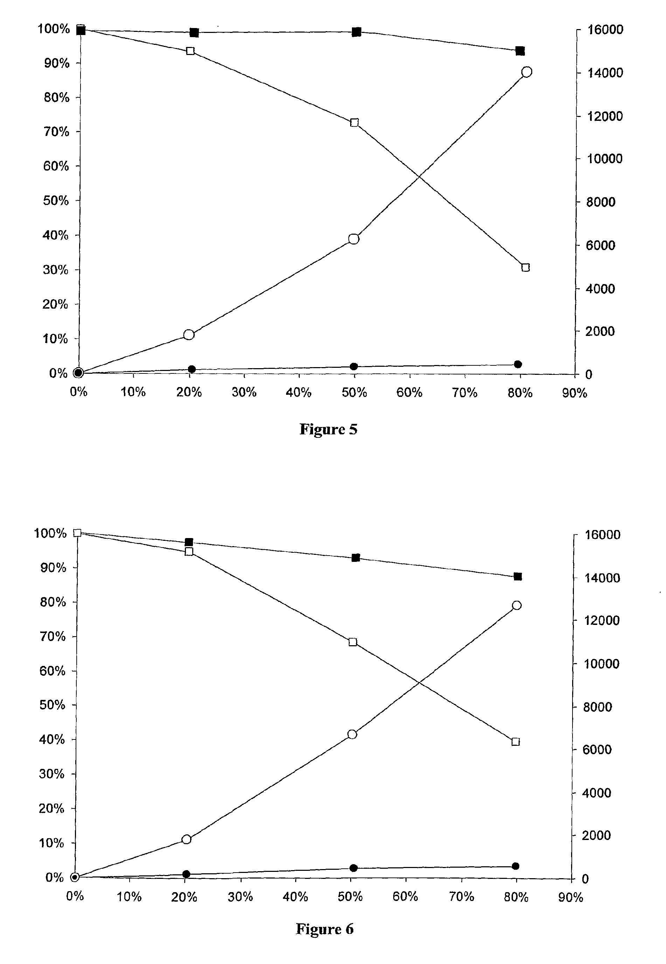

Figure 5 : Rendement et concentration résiduelle en glycérol après culture en mode chemostat de la souche I-3887 et de la souche NCYC 996, en fonction de la proportion de glycérol dans le mélange de glycérol et de sucre du milieu d'alimentation.

L'axe des ordonnées à gauche indique le rendement de la souche en pourcentage par rapport au rendement de la souche NCYC 996 cultivée en l'absence de glycérol. L'axe des ordonnées à droite indique la concentration résiduelle en glycérol dans le milieu débarrassé des levures (en ppm). L'axe des abscisses indique la proportion de glycérol dans le mélange de glycérol et de sucre en équivalent saccharose. Le rendement de production est représenté par des carrés et la concentration résiduelle en glycérol par des cercles. Les résultats de la souche non mutée NCYC 996 (témoin) figurent en blanc et ceux de la souche mutée correspondante I-3887 en noir.

Figure 6 : Rendement et concentration résiduelle en glycérol après culture en mode chemostat de la souche I-3888 et de la souche NCYC 995, en fonction de la proportion de glycérol dans le mélange de glycérol et de sucre du milieu d'alimentation.

L'axe des ordonnées à gauche indique le rendement de la souche en pourcentage par rapport au rendement de la souche NCYC 995 cultivée en l'absence de glycérol. L'axe des ordonnées à droite indique la concentration résiduelle en glycérol dans le milieu débarrassé des levures (en ppm). L'axe des abscisses indique la proportion de glycérol dans le mélange de glycérol et de sucre en équivalent saccharose. Le rendement de production est représenté par des carrés et la concentration résiduelle en glycérol par des cercles. Les résultats de la souche non mutée NCYC 995 (témoin) figurent en blanc et ceux de la souche mutée correspondante I-3888 en noir.

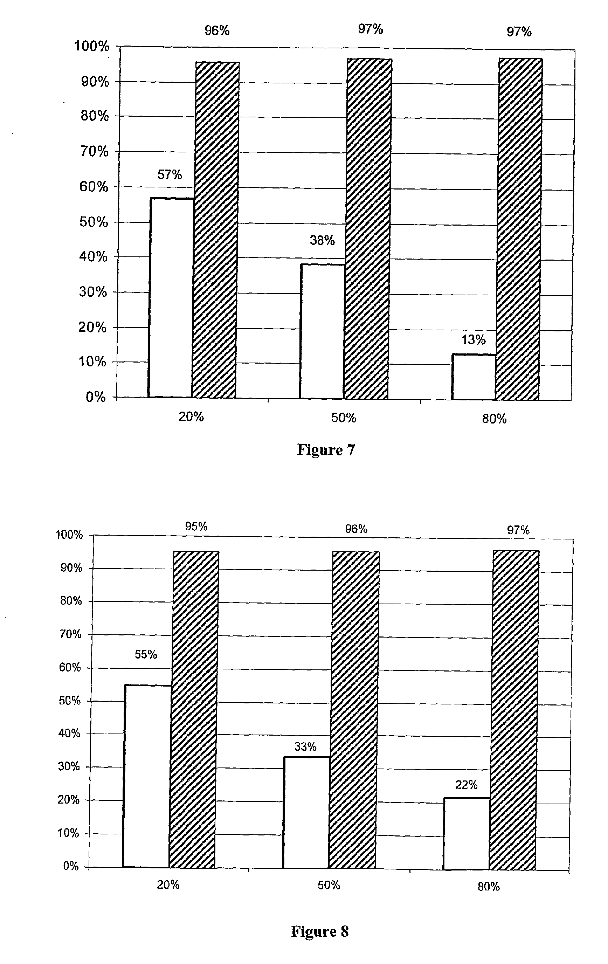

Figure 7 : Pourcentage de glycérol consommé après culture en mode chemostat de la souche I-3887 et de la souche NCYC 996, en fonction de la proportion de glycérol dans le mélange de glycérol et de sucre du milieu d'alimentation.

Le pourcentage de glycérol consommé (en ordonnées) est donné en fonction de la proportion de glycérol dans le mélange de glycérol et de sucre en équivalent saccharose (en abscisses). Le pourcentage de glycérol consommé par la souche non mutée NCYC 996 (témoin) est représenté en blanc et celui de la souche mutée correspondante I-3887 en hachuré.

Figure 8 : Pourcentage de glycérol consommé après culture en mode chemostat de la souche I-3888 et de la souche NCYC 995, en fonction de la proportion de glycérol dans le mélange de glycérol et de sucre du milieu d'alimentation.

Le pourcentage de glycérol consommé (en ordonnées) est donné en fonction de la proportion de glycérol dans le mélange de glycérol et de sucre en équivalent saccharose (en abscisses). Le pourcentage de glycérol consommé par la souche non mutée NCYC 995 (témoin) est représenté en blanc et celui de la souche mutée correspondante I-3888 en hachuré.

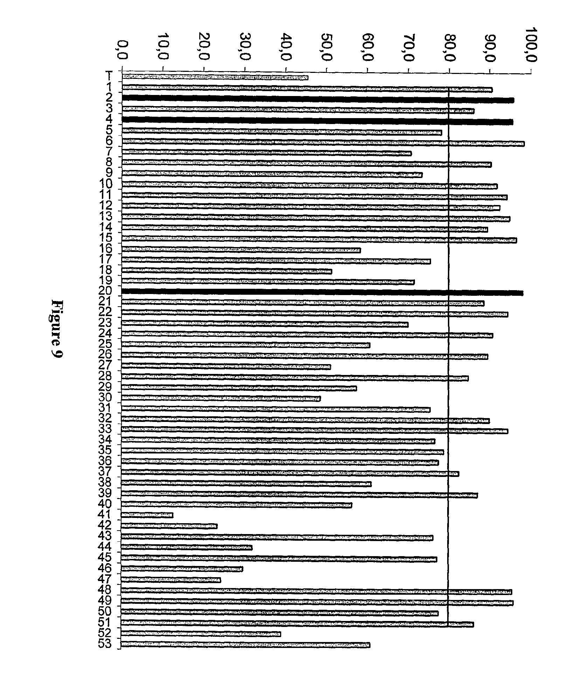

Figure 9 : Pourcentage de glycérol consommé après culture en mode semi-continu de variants naturels en présence d'un mélange de glycérol et de sucre comprenant 20 % de glycérol en équivalent saccharose.

Le pourcentage de glycérol consommé (en ordonnées) est indiqué pour différents variants naturels testés (1 à 53). Le pourcentage de glycérol consommé de la souche non mutée dont sont issus les variants naturels (notée T) est également indiqué.

### DESCRIPTION DETAILLEE DE L'INVENTION

Il est décrit ici une souche de Saccharomyces, caractérisée en ce qu'en présence d'un milieu de culture comprenant un mélange de glycérol et de sucre, en condition aérobie, elle consomme au moins 80 % du glycérol dudit mélange, ledit mélange comprenant une proportion de glycérol d'au moins 20 % en équivalent saccharose, la somme des proportions de glycérol et de sucre dudit mélange étant égale à 100 % en équivalent saccharose.

De manière surprenante et inattendue, les Inventeurs ont obtenu des souches de levure de *Saccharomyces* capables de consommer au moins 80 % du glycérol d'un mélange de glycérol et de sucre, simultanément à leur consommation de sucre, en condition aérobie, dans une culture semi-continue ou continue.

Les Inventeurs ont en effet montré que des levures de boulangerie industrielles classiques, telles que les souches déposées à la NCYC (National Collection of Yeast Cultures) sous les numéros NCYC995 et NCYC996, ne consomment le glycérol que partiellement en présence d'un mélange de glycérol et de sucre en culture semi-continue et/ou en culture continue (voir exemple 2). En fin de culture, le milieu de culture débarrassé des levures contient alors une forte quantité résiduelle de glycérol qui s'est accumulé au cours de la culture.

Les souches selon l'invention sont caractérisées par le fait qu'en présence d'un milieu de culture comprenant un mélange de glycérol et de sucre, elles consomment au moins 80 % du glycérol dudit mélange, le pourcentage étant donné en poids/poids. En d'autres termes, la souche selon l'invention consomme au moins 80 % de la masse totale de glycérol présente dans le mélange de glycérol et de sucre.

La présente invention a particulièrement pour objet une souche de *Saccharomyces,* caractérisée en ce qu'en présence d'un milieu de culture comprenant un mélange de glycérol et de sucre, en condition aérobie, elle consomme au moins 80 % du glycérol dudit mélange, ledit mélange comprenant une proportion de glycérol de 20 % en équivalent saccharose, la somme des proportions de glycérol et de sucre dudit mélange étant égale à 100 % en équivalent saccharose.

La détermination de la consommation en glycérol peut être effectuée par une mesure de la quantité résiduelle de glycérol dans le milieu de culture, après une culture semi-continue, ou par une mesure de la quantité résiduelle de glycérol dans le milieu soutiré dans le cas d'une culture continue.

Dans le cas d'une culture semi-continue, la mesure de la quantité résiduelle de glycérol dans le milieu de culture est effectuée à la fin de la culture.

Par « fin de la culture », on désigne le moment où l'alimentation en sucre est arrêtée.

La durée de la culture est en particulier inférieure ou égale à la durée d'une culture de la même souche sur un mélange comportant 100 % de sucre en équivalent saccharose.

La durée de la culture est en particulier inférieure à 24h.

La durée de la culture est en particulier supérieure à 20h.

La durée de la culture est par exemple de 22h.

Dans le cas d'une culture continue, la quantité résiduelle de glycérol est mesurée à n'importe quel instant, une fois que le régime permanent est atteint.

Le régime permanent est atteint lorsque les concentrations dans le fermenteur sont stables pendant au moins 3 temps de séjour, le temps de séjour étant le rapport 1/[taux de dilution].

De façon conventionnelle, les conditions de culture sont telles que le métabolisme de la souche de levure est essentiellement respiratoire et/ou telles qu'il n'y a essentiellement pas de production d'éthanol.

Le terme « essentiellement » signifie que ces conditions sont vérifiées sur la durée totale de la culture, mais qu'il est possible que ponctuellement, ces conditions ne soient pas vérifiées en mode semi-continu, par exemple pendant les premières heures de la culture, notamment pendant une durée comprise de 1/4 à 1/3 de la durée de culture, et en particulier pendant les 5 premières heures d'une culture en mode semi-continu.

Dans un mode chemostat, les conditions de culture sont telles que le métabolisme de la souche de levure est respiratoire et/ou telles qu'il n'y a pas de production d'éthanol lorsque le régime permanent est atteint.

L'expression « le métabolisme de la souche de levure est essentiellement respiratoire » signifie que le carbone est oxydé, et non fermenté, sur la durée totale de la culture, une partie du carbone pouvant être fermentée de manière transitoire.

L'expression « il n'y a essentiellement pas de production d'éthanol » signifie que la concentration en éthanol en fin de culture est inférieure à 1 %, de préférence inférieure à 0,1 %, plus préférentiellement inférieure à 0,01 %, plus préférentiellement inférieure à 0,001 %.

L'homme du métier sait comment régler les paramètres de culture d'une souche de levure donnée, afin que les conditions de culture soient telles que le métabolisme de ladite souche est essentiellement respiratoire et/ou telles qu'il n'y a essentiellement pas de production d'éthanol.

La quantité résiduelle de glycérol est de préférence mesurée dans le milieu de culture débarrassé des levures.

La quantité résiduelle de glycérol dans le milieu de culture est par exemple mesurée par chromatographie HPLC ou CPG, dosage enzymatique ou toute autre méthode appropriée.

Les souches de levure selon l'invention sont donc particulièrement intéressantes, car elles sont capables de consommer le glycérol en continu, sur une durée de production compatible avec une exploitation industrielle.

L'expression « capables de consommer le glycérol en continu » signifie qu'il n'y a essentiellement pas d'accumulation de glycérol sur la durée totale de culture, mais qu'une accumulation ponctuelle reste possible si le métabolisme de la levure n'est pas essentiellement respiratoire et/ou s'il y a une production d'alcool.

Par l'expression « mélange de glycérol et de sucre », on désigne un mélange constitué de glycérol et de sucre dans des proportions variables, la somme des proportions de glycérol et de sucre dudit mélange étant égale à 100 % en équivalent saccharose.

Le mélange de glycérol et de sucre constitue la seule source de sucre et de glycérol dans le milieu de culture.

Le mélange de glycérol et de sucre est, de préférence, préparé avant son introduction dans le milieu de culture.

Le glycérol, appelé également glycérine ou propan-1,2,3-triol est un polyol. Le glycérol pur se présente sous la forme d'un liquide transparent et visqueux et il est soluble dans l'eau.

Le glycérol selon l'invention peut être utilisé sous forme pure et/ou sous la forme d'un produit contenant du glycérol, et/ou être apporté sous toute forme capable de donner du glycérol.

Le sucre est de préférence un sucre immédiatement assimilable par la levure, notamment un sucre simple de type glucose, fructose, saccharose, et/ou un mélange de ces sucres.

Le sucre peut être apporté sous la forme de sirops de glucose et/ou de sirops de fructose et/ou d'hydrolysats d'amidon et/ou sous la forme de mélasses et/ou sous la forme d'une substance capable de donner des sucres assimilables par la souche de levure et/ou sous la forme d'un mélange de ceux-ci.

Le sucre est de préférence apporté sous la forme de mélasses.

Le mélange de glycérol et de sucre constitue au moins 90 % de la source carbonée pour la levure, de préférence au moins 95 % de la source carbonée, plus préférentiellement au moins 98 % de la source carbonée. La source carbonée dans le milieu de culture, autre que le mélange de glycérol et de sucre, comprend par exemple des acides aminés, des lipides, des acides organiques naturellement présents dans les mélasses ou les hydrolysats bruts d'origine agricole.

L'expression «la somme des proportions de glycérol et de sucre dudit mélange étant égale à 100 % en équivalent saccharose » signifie que lorsque la proportion de glycérol exprimée en équivalent saccharose est de x %, la proportion de sucre dans le mélange exprimée en équivalent saccharose est de (100-x) %.

L'équivalence est déterminée ici par rapport au métabolisme de la glycolyse, selon lequel une mole de saccharose est convertie en une mole de fructose et une mole de glucose qui sont elles-mêmes converties en quatre moles de pyruvate (cf. Principes de la Biochimie, Lehninger, 1994).

Une mole de glucose est donc convertie en deux moles de pyruvate et une mole de fructose en deux moles de pyruvate.

Selon ce même métabolisme, une mole de glycérol est convertie en une mole de pyruvate.

Ainsi, quatre moles de glycérol correspondent à une mole d'équivalent saccharose.

Deux moles de glucose correspondent à une mole d'équivalent saccharose.

Deux moles de fructose correspondent à une mole d'équivalent saccharose.

1,076 g de glycérol correspondent ici à un gramme d'équivalent saccharose.

Par exemple, un kilogramme d'un mélange contenant au final 250 g/kg d'équivalent saccharose et une proportion de 20 % de glycérol en équivalent saccharose est donc constitué de 50 g d'équivalent saccharose sous la forme de glycérol et de 200 g d'équivalent saccharose sous la forme de sucre. Etant donnée une mélasse brute ayant une teneur en sucre de 500 g/kg en équivalent saccharose, pour préparer un kilogramme de ce mélange, on procède d'abord au mélange de 50 *1.076 g = 54 g de glycérol pur (ce qui correspond à 50 g d'équivalent saccharose sous la forme de glycérol) et de 400 g de ladite mélasse brute (ce qui correspond à 200 g d'équivalent saccharose sous la forme de sucre), puis de l'eau pure est ajoutée pour atteindre une masse finale de mélange de 1 kilogramme.

Le milieu de culture est appelé ici milieu de culture frais lorsqu'il n'a pas été mis en présence de souches de levure.

Le milieu de culture est appelé ici milieu réactionnel lorsqu'il a été mis en présence de souches de levure.

La souche de levure selon l'invention est mise en présence du milieu de culture en condition aérobie.

De préférence, la souche de levure selon l'invention est mise en présence du milieu de culture dans un fermenteur adapté à la production de levure. Le volume du fermenteur peut varier de quelques millilitres à plusieurs mètres cube.

Le fermenteur est également appelé réacteur par la suite.

Le fermenteur est de préférence adapté pour une culture en condition aérobie.

La souche de levure est de préférence mise en présence du milieu de culture dans le cadre d'une culture en mode semi-continu et/ou en mode continu.

Par l'expression « culture en mode semi-continu » ou « culture semi-continue » ou « mode semi-continu », appelée également « fed-batch », on désigne ici une culture dans un fermenteur qui est alimenté progressivement par le milieu de culture, mais dont aucun volume de milieu n'est soutiré. Dans un tel procédé, le volume de culture est variable (et généralement croissant) dans le fermenteur. Le débit d'alimentation dans une culture en mode semi-continu est constant ou variable.

Un exemple de culture semi-continue est une culture réalisée dans les conditions décrites dans le livre de référence « Yeast Technology », Chapitre 6, 2ème édition, 1991, G. Reed et T.W. Nagodawithana, publié par Van Nostrand Reinhold, ISBN 0-442-31892-8.

Par l'expression « culture en mode continu » ou « culture continue » ou « mode continu », on désigne ici une culture dans un fermenteur au cours de laquelle le fermenteur est alimenté en milieu de culture frais et dont une partie du milieu réactionnel est soutirée. Dans une culture en mode continu, le volume de culture dans le fermenteur est variable ou constant.

Selon un mode de réalisation particulier de la culture en mode continu, le débit d'alimentation et le débit de soutirage sont égaux.

Un exemple de culture continue est une culture réalisée dans les conditions décrites dans le livre de référence «Yeast Technology », Chapitre 6, 2ème édition, 1991, G. Reed et T.W. Nagodawithana, publié par Van Nostrand Reinhold, ISBN 0-442-31892-8.

La culture en mode chemostat est une forme particulière de culture continue.

Dans une culture en mode chemostat, les différents paramètres tels que le taux d'oxygène dissous, les concentrations en nutriments, le pH, la densité cellulaire, sont maintenus constants.

Le milieu de culture comprend comme constituants le mélange de glycérol et de sucre et les autres éléments nécessaires à la croissance des levures.

Les autres éléments nécessaires à la croissance des levures sont les suivants : au moins une source d'azote, au moins une source de soufre, au moins une source de phosphore, au moins une source de vitamines et/ou au moins une source de minéraux.

Ces autres éléments sont apportés en quantités suffisantes pour ne pas constituer un facteur limitant de la croissance des levures et pour maintenir un métabolisme essentiellement respiratoire.

La source d'azote est par exemple apportée sous la forme de sulfate d'ammonium, hydroxyde d'ammonium, di-ammonium phosphate, ammoniac, urée et/ou une combinaison de ceux-ci.

La source de soufre est par exemple apportée sous la forme de sulfate d'ammonium, sulfate de magnésium, acide sulfurique et/ou une combinaison de ceux-ci.

La source de phosphore est par exemple apportée sous la forme d'acide phosphorique, phosphate de potassium, di-ammonium phosphate, mono-ammonium phosphate, et/ou une combinaison de ceux-ci.

La source de vitamines est par exemple apportée sous la forme de mélasse, hydrolysat de levure, solution de vitamine pure ou d'un mélange de vitamines pures et/ou une combinaison de ceux-ci.

La source de vitamines apporte à la levure l'ensemble des vitamines dans des quantités au moins équivalentes à celles recommandées dans les ouvrages de référence. Plusieurs sources de vitamines peuvent être associées.

La source de minéraux est par exemple apportée sous la forme de mélasse, un mélange de sels minéraux et/ou leur combinaison.

La source de minéraux apporte à la levure l'ensemble des macroéléments et oligoéléments dans des quantités au moins équivalentes à celles recommandées dans les ouvrages de référence. Plusieurs sources de minéraux peuvent être associées.

Une même substance peut apporter plusieurs éléments différents.

Les souches de levure selon l'invention sont en particulier choisies parmi les souches permettant de produire de la levure de panification, de la levure de brasserie, de la levure d'oenologie, de la levure pour production d'alcool, et/ou de la levure pour la production de protéines recombinantes, par exemple pour la production de protéines thérapeutiques.

La consommation en glycérol d'une souche de *Saccharomyces* peut, par exemple, être mesurée dans les conditions décrites dans l'exemple 2, afin de déterminer si ladite souche, en présence d'un milieu de culture comprenant un mélange de glycérol et de sucre, en condition aérobie, consomme au moins 80 % du glycérol du mélange, ledit mélange comprenant une proportion de glycérol de 20 % en équivalent saccharose, la somme des proportions de glycérol et de sucre dudit mélange étant égale à 100 % en équivalent saccharose.

Il est également décrit ici une souche de *Saccharomyces*, caractérisée en ce qu'en présence d'un milieu de culture comprenant un mélange de glycérol et de sucre, en condition aérobie, elle consomme au moins 80 % du glycérol dudit mélange, ledit mélange comprenant une proportion de glycérol de 30 % en équivalent saccharose, la somme des proportions de glycérol et de sucre dudit mélange étant égale à 100 % en équivalent saccharose.

Il est également décrit ici une souche de *Saccharomyces*, caractérisée en ce qu'en présence d'un milieu de culture comprenant un mélange de glycérol et de sucre, en condition aérobie, elle consomme au moins 80 % du glycérol dudit mélange, ledit mélange comprenant une proportion de glycérol de 40 % en équivalent saccharose, la somme des proportions de glycérol et de sucre dudit mélange étant égale à 100 % en équivalent saccharose.

Il est en particulier décrit ici une souche telle que définie ci-dessus, caractérisée en ce qu'elle consomme au moins 85 % du glycérol du mélange, de préférence au moins 90 % du glycérol du mélange, plus préférentiellement au moins 95 % du glycérol du mélange, encore plus préférentiellement au moins 98 % du glycérol du mélange.

Il est aussi décrit ici une souche telle que définie ci-dessus, caractérisée en ce que ledit mélange comprend une proportion de glycérol d'au moins 30 % en équivalent saccharose, de préférence au moins 40 % en équivalent saccharose, plus préférentiellement au moins 50 % en équivalent saccharose, encore plus préférentiellement au moins 60 % en équivalent saccharose, encore plus préférentiellement au moins 70 % en équivalent saccharose, encore plus préférentiellement au moins 80 % en équivalent saccharose, encore plus préférentiellement au moins 90 % en équivalent saccharose.

Il est aussi décrit ici une souche telle que définie ci-dessus, caractérisée en ce que la proportion de glycérol dans ledit mélange est au plus de 95 % en équivalent saccharose, de préférence au plus 90 % en équivalent saccharose, plus préférentiellement au plus 85 % en équivalent saccharose.

La présente invention a particulièrement pour objet une souche telle que définie ci-dessus, caractérisée en ce que la proportion de glycérol dans ledit mélange est comprise de 20 % à 90 % en équivalent saccharose, de préférence 20 % à 80 % en équivalent saccharose, plus préférentiellement de 30 % à 70 % en équivalent saccharose, encore plus préférentiellement de 30 % à 60 % en équivalent saccharose.

Il est décrit ici une souche telle que définie ci-dessus, caractérisée en ce qu'elle est choisie parmi les espèces *cerevisiae*, *boulardii*, *carlsbergensis* et *uvarum.*

Une souche préférée selon l'invention est une souche de *Saccharomyces cerevisiae*.

Il est décrit ici une souche telle que définie ci-dessus, caractérisée en ce qu'elle est choisie parmi un variant naturel et/ou une souche génétiquement modifiée.

Le terme « variant naturel » désigne ici une souche obtenue par croisement et/ou hybridation de souches de levure et/ou obtenue par mutation spontanée et/ou par mutagenèse aléatoire, par exemple suite à une exposition à des conditions de stress, à un traitement UV ou à un traitement avec des agents mutagènes.

Le patrimoine génétique d'un variant naturel n'a pas été modifié par génie génétique.

Deux méthodes de mutagenèse aléatoire peuvent par exemple être utilisées pour obtenir des variants naturels (voir également l'exemple 3 ci-dessous).

La première méthode consiste en une sélection des variants naturels sur la base de leur consommation en glycérol et de leur rendement, directement après une exposition à un rayonnement UV suivie d'un isolement de clones.

La deuxième méthode comprend :
- une première étape d'exposition à un rayonnement UV,
- une deuxième étape de culture en mode chemostat en présence d'un mélange de sucre et de glycérol,
- une troisième étape d'isolement de clones, et
- une quatrième étape de sélection des variants naturels sur la base de leur consommation en glycérol et de leur rendement.

Cette deuxième méthode permet d'enrichir la culture en variants naturels consommant le glycérol en présence de sucre, avant de commencer l'étape de sélection.

L'invention a ainsi particulièrement pour objet la souche déposée le 3 mars 2009 auprès de la CNCM sous le numéro CNCM I-4129, la souche déposée le 3 mars 2009 auprès de la CNCM sous le numéro CNCM I-4130 et la souche déposée le 3 mars 2009 auprès de la CNCM sous le numéro CNCM I-4131.

Ces trois souches ont été obtenues par mutagenèse aléatoire suite à un traitement UV et sélection sur la base de leur consommation en glycérol et leur rendement (voir exemple 3).

La présente invention a pour objet un variant naturel tel que défini ci-dessus, caractérisé en ce qu'il s'agit de la souche numéro CNCM 1-4129.

En culture semi-continue, dans un milieu de culture contenant un mélange de glycérol et de sucre avec une proportion de glycérol de 20 % en équivalent saccharose, la souche de levure CNCM I-4129 consomme plus de 95 % du glycérol du mélange.

En culture continue, dans un milieu de culture contenant un mélange de glycérol et de sucre, la souche de levure CNCM I-4129 consomme plus de 80 % du glycérol du mélange, lorsque la proportion de glycérol dans ledit mélange varie de 20 % à 50 %.

La présente invention a pour objet un variant naturel tel que défini ci-dessus, caractérisé en ce qu'il s'agit de la souche numéro CNCM I-4130.

En culture semi-continue, dans un milieu de culture contenant un mélange de glycérol et de sucre avec une proportion de glycérol de 20 % en équivalent saccharose, la souche de levure CNCM I-4130 consomme plus de 95 % du glycérol du mélange.

En culture continue, dans un milieu de culture contenant un mélange de glycérol et de sucre, la souche de levure CNCM I-4130 consomme plus de 80 % du glycérol du mélange, lorsque la proportion de glycérol dans ledit mélange varie de 20 % à 50 %.

La présente invention a pour objet un variant naturel tel que défini ci-dessus, caractérisé en ce qu'il s'agit de la souche numéro CNCM I-4131.

En culture semi-continue, dans un milieu de culture contenant un mélange de glycérol et de sucre avec une proportion de glycérol de 20 % en équivalent saccharose, la souche de levure CNCM I-4131 consomme plus de 95 % du glycérol du mélange.

En culture continue, dans un milieu de culture contenant un mélange de glycérol et de sucre, la souche de levure CNCM I-4131 consomme plus de 80 % du glycérol du mélange, lorsque la proportion de glycérol dans ledit mélange varie de 20 % à 50 %.

Par l'expression « souche génétiquement modifiée » ou souche OGM, on désigne une souche dont le patrimoine génétique a été modifié par génie génétique.

Une souche génétiquement modifiée peut avoir subi une modification de son ADN endogène, par exemple par une mutation ponctuelle, partielle ou totale d'un gène. La mutation peut consister en une insertion, délétion et/ou substitution d'au moins un nucléotide.

La mutation induit de préférence une modification du produit du gène après transcription et/ou traduction. La mutation peut notamment entraîner une troncature du produit du gène, une modification d'au moins un acide aminé dans la séquence du produit du gène et/ou un décalage du cadre de lecture lors de la transcription.

Une souche génétiquement modifiée peut comporter un ADN exogène, intégré ou non dans au moins un chromosome de la levure et/ou dans au moins un plasmide de la levure.

Une souche génétiquement modifiée préférée comporte un ADN exogène intégré dans au moins un chromosome de la levure.

L'ADN exogène peut être un fragment d'ADN, - par exemple un fragment de gène, une cassette d'expression complète ou partielle -, et/ou un plasmide.

Lorsque la souche génétiquement modifiée comporte un ADN exogène intégré, il s'agit d'une intégration unique ou d'une intégration multicopies.

Il est décrit ici une souche telle que définie ci-dessus, caractérisée en ce qu'il s'agit d'une souche génétiquement modifiée au niveau d'au moins un gène impliqué dans au moins une voie métabolique du glycérol.

Il est décrit ici une souche telle que définie ci-dessus, caractérisée en ce qu'il s'agit d'une souche génétiquement modifiée au niveau d'au moins un gène impliqué dans le catabolisme du glycérol ou dans la régulation du catabolisme du glycérol.

Il est en particulier décrit ici une souche telle que définie ci-dessus, caractérisée en ce qu'il s'agit d'une souche génétiquement modifiée au niveau d'au moins un gène choisi parmi les gènes STL1, GUP1, GUP2, GUT1, GUT2, FPS1 et/ou ADR1 et/ou un gène impliqué dans la répression par le glucose et/ou d'une combinaison de ceux-ci.

Une souche génétiquement modifiée selon l'invention présente notamment une augmentation de l'expression d'un gène et / ou une diminution de l'expression d'un gène et / ou une expression constitutive et / ou une expression différentielle d'un gène, et/ou l'expression d'un gène absent dans la souche de levure d'origine.

Il est en particulier décrit ici une souche telle que définie ci-dessus, caractérisée en ce qu'il s'agit d'une souche génétiquement modifiée au niveau d'au moins un gène choisi parmi les gènes STL1, GUP1, GUP2, GUT1, GUT2 et/ou d'une combinaison de ceux-ci.

La modification des souches selon l'invention est effectuée en utilisant les techniques bien connues de l'homme du métier (voir Molecular Cloning, 3rd édition, Sambrook et Russel).

Une souche de levure génétiquement modifiée comme décrit ici consomme de préférence plus de 90 % du glycérol du mélange de glycérol et de sucre.

Le gène STL1 code la perméase au glycérol qui permet en particulier l'entrée du glycérol dans la cellule.

Une souche selon l'invention génétiquement modifiée au niveau du gène STL1 exprime le gène STL1 en présence de sucre.

Une souche génétiquement modifiée au niveau du gène STL1 préférée selon l'invention surexprime le gène STL1 en présence de sucre par rapport à la souche non mutée correspondante.

Le gène STL1 dans la souche génétiquement modifiée selon l'invention est par exemple placé sous contrôle d'un promoteur constitutif ou d'un promoteur induit par le glucose.

Une souche génétiquement modifiée au niveau du gène STL1 préférée selon l'invention présente un taux de transcription du gène STL1 multiplié au moins par 100 par rapport à celui de la souche non mutée correspondante, de préférence multiplié au moins par 200, plus

préférentiellement au moins par 250, le taux de transcription étant calculé après une PCR quantitative sur de l'ADNc obtenu par transcription inverse.

Le gène GUP1 code pour une protéine membranaire impliquée dans le transport actif du glycérol dans la cellule.

Une souche génétiquement modifiée au niveau de GUP1 exprime le gène GUP1 en présence de sucre.

Le gène GUP2 code pour une protéine membranaire impliquée dans le transport actif du glycérol dans la cellule.

Une souche génétiquement modifiée au niveau de GUP2 exprime le gène GUP2 en présence de sucre.

Le gène FPS1 ou YLL043W code pour une protéine membranaire de transport permettant en particulier l'export du glycérol.

Une souche génétiquement modifiée au niveau de FPS1 n'exprime pas le gène FPS1, par exemple suite à une délétion du gène.

Le gène ADR1 ou YDR216W code pour un activateur de transcription impliqué dans l'expression de gènes impliqués dans les voies d'utilisation de l'éthanol, du glycérol et des acides gras. De tels gènes sont notamment régulés par la répression au glucose.

Une souche génétiquement modifiée au niveau de ADR1 exprime le gène ADR1 en présence de sucre.

Le gène GUT1 ou YHL032C code pour une glycérol kinase qui convertit le glycérol en glycérol-3-phosphate.

Une souche génétiquement modifiée au niveau de GUT1 exprime le gène GUT1 en présence de sucre.

Le gène GUT2 ou YIL155C code pour la glycérol-3-phosphate déshydrogénase qui convertit le glycérol-3-phosphate en DHAP (dihydroxyacétone-phosphate).

Une souche génétiquement modifiée au niveau de GUT2 exprime le gène GUT2 en présence de sucre.

Une souche est par exemple modifiée par transformation avec une cassette d'expression permettant l'expression d'un gène tel que défini ci-dessus, en particulier en présence de sucre.

La cassette d'expression comprend un promoteur, un gène d'intérêt et un terminateur. Le terminateur est une séquence artificielle ou une séquence terminatrice de levure.

Le promoteur est un promoteur constitutif ou inductible.

Un promoteur préféré est le promoteur pADH1 inductible par le glucose.

La présente invention a pour objet une souche telle que définie ci-dessus, caractérisée en ce qu'il s'agit de la souche déposée le 20 décembre 2007 auprès de la CNCM sous le numéro CNCM I-3886.

La souche de levure numéro CNCM I-3886 a été obtenue par intégration de la cassette d'expression représentée à la figure 2 dans le génome de la souche déposée à la CNCM sous le numéro CNCM I-3399.

La souche de levure numéro CNCM I-3886 exprime le gène STL1 fortement en présence de sucre (taux d'ARNm plus de 270 fois supérieur à celui de la souche non mutée I-3399).

En culture semi-continue, dans un milieu de culture contenant un mélange de glycérol et de sucre avec une proportion de glycérol de 20 % en équivalent saccharose, la souche de levure CNCM I-3886 consomme plus de 95 % du glycérol du mélange.

En culture continue, dans un milieu de culture contenant un mélange de glycérol et de sucre, la souche de levure CNCM I-3886 consomme plus de 90 % du glycérol du mélange, lorsque la proportion de glycérol dans ledit mélange varie de 20 % à 80 %.

La présente invention a pour objet une souche telle que définie ci-dessus, caractérisée en ce qu'il s'agit de la souche déposée le 20 décembre 2007 auprès de la CNCM sous le numéro CNCM I-3887.

La souche de levure numéro CNCM I-3887 a été obtenue par intégration de la cassette d'expression représentée à la figure 2 dans le génome de la souche déposée à la NCYC (*National Collection of Yeast Cultures*) sous le numéro NCYC 996.

La souche de levure numéro CNCM I-3887 exprime le gène STL1 fortement en présence de sucre (taux d'ARNm plus de 270 fois supérieur à celui de la souche non mutée NCYC 996).

En culture semi-continue, dans un milieu de culture contenant un mélange de glycérol et de sucre avec une proportion de glycérol de 20 % en équivalent saccharose, la souche de levure CNCM I-3887 consomme plus de 95 % du glycérol du mélange.

En culture continue, dans un milieu de culture contenant un mélange de glycérol et de sucre, la souche de levure CNCM I-3887 consomme plus de 90 % du glycérol du mélange, lorsque la proportion de glycérol dans ledit mélange varie de 20 % à 80 %.

La présente invention a pour objet une souche telle que définie ci-dessus, caractérisée en ce qu'il s'agit de la souche déposée le 20 décembre 2007 auprès de la CNCM sous le numéro CNCM I-3888.

La souche de levure numéro CNCM I-3888 a été obtenue par intégration de la cassette d'expression représentée à la figure 2 dans le génome de la souche déposée à la NCYC (*National Collection of Yeast Cultures*) sous le numéro NCYC 995.

La souche de levure numéro CNCM I-3888 exprime donc le gène STL1 fortement en présence de sucre (taux d'ARNm plus de 300 fois supérieur à celui de la souche non mutée NCYC 995).

En culture semi-continue, dans un milieu de culture contenant un mélange de glycérol et de sucre avec une proportion de glycérol de 20 % en équivalent saccharose, la souche de levure CNCM I-3888 consomme plus de 95 % du glycérol du mélange.

En culture continue, dans un milieu de culture contenant un mélange de glycérol et de sucre, la souche de levure CNCM I-3888 consomme plus de 90 % du glycérol du mélange, lorsque la proportion de glycérol dans ledit mélange varie de 20 % à 80 %.

Il est décrit ici une souche telle que définie ci-dessus, caractérisée en ce qu'il s'agit de la souche déposée le 3 mars 2009 auprès de la CNCM sous le numéro CNCM I-4132.

La souche de levure CNCM I-4132 a été obtenue par intégration de la cassette d'expression représentée à la figure 2 dans le génome de la souche déposée le 4 septembre 2008 à la CNCM sous le numéro I-4072.

La souche de levure numéro CNCM I-4132 exprime donc le gène STL1 fortement en présence de sucre (taux d'ARNm plus de 200 fois supérieur à celui de la souche non mutée I-4072).

En culture semi-continue, dans un milieu de culture contenant un mélange de glycérol et de sucre avec une proportion de glycérol de 20 % en équivalent saccharose, la souche de levure CNCM I-4132 consomme plus de 80 % du glycérol du mélange.

Il est décrit ici une souche telle que définie ci-dessus, caractérisée en ce qu'il s'agit de la souche déposée le 3 mars 2009 auprès de la CNCM sous le numéro CNCM I-4133.

La souche de levure CNCM I-4133 a été obtenue par intégration de la cassette d'expression représentée à la figure 2 dans le génome de la souche déposée le 4 septembre 2008 à la CNCM sous le numéro I-4071.

La souche de levure numéro CNCM I-4133 exprime le gène STL1 fortement en présence de sucre (taux d'ARNm plus de 200 fois supérieur à celui de la souche non mutée I-4071).

En culture semi-continue, dans un milieu de culture contenant un mélange de glycérol et de sucre avec une proportion de glycérol de 20 % en équivalent saccharose, la souche de levure CNCM I-4133 consomme plus de 80 % du glycérol du mélange.

Il est également décrit ici pour objet une souche de *Saccharomyces* dérivée de la souche CNCM I-3886, de la souche CNCM I-3887, de la souche CNCM I-3888, de la souche CNCM I-4132, de la souche CNCM I-4133, de la souche CNCM I-4129, de la souche CNCM I-4130 ou de la souche CNCM I-4131, caractérisée en ce qu'en présence d'un milieu de culture comprenant un mélange de glycérol et de sucre, elle consomme au moins 80 % du glycérol dudit mélange, ledit mélange comprenant une proportion de glycérol d'au moins 20 % en équivalent saccharose, la somme des proportions de glycérol et de sucre dudit mélange étant égale à 100 % en équivalent saccharose.

Il est également décrit ici une souche de *Saccharomyces* dérivée de la souche CNCM I-3886, de la souche CNCM I-3887, de la souche CNCM I-3888, de la souche CNCM I-4132, de la souche CNCM I-4133, de la souche CNCM I-4129, de la souche CNCM I-4130 ou de la souche CNCM I-4131, caractérisée en ce qu'en présence d'un milieu de culture comprenant un mélange de glycérol et de sucre, elle consomme au moins 80 % du glycérol dudit mélange, ledit mélange comprenant une proportion de glycérol de 20 % en équivalent saccharose, la somme des proportions de glycérol et de sucre dudit mélange étant égale à 100 % en équivalent saccharose.

Par l'expression « souche dérivée », on désigne une souche dérivée par toute transformation quelle qu'elle soit, comme par exemple par un ou plusieurs croisements et/ou par mutation et/ou par transformation génétique.

La présente invention a également pour objet l'utilisation d'une souche de levure de l'invention, pour la production de levures, en présence d'un mélange de glycérol et de sucre, ledit mélange comprenant une proportion de glycérol d'au moins 5 % en équivalent saccharose, la somme des proportions de glycérol et de sucre dudit mélange étant égale à 100 % en équivalent saccharose.

La présente invention a notamment pour objet l'utilisation d'une souche de levure de l'invention, pour la production industrielle de levures, en présence d'un mélange de glycérol et de sucre.

La présente invention a pour objet l'utilisation d'une souche de levure de l'invention, pour la production de levures, la production étant effectuée selon un schéma classique bien connu de l'homme du métier, mais réalisée en condition aérobie et en utilisant un mélange de glycérol et de sucre comme source de carbone.

La présente invention a également pour objet l'utilisation d'un mélange de glycérol et de sucre pour la production de levures à partir d'au moins une souche de *Saccharomyces* de l'invention, caractérisée en ce que ladite souche consomme au moins 80 % du glycérol dudit mélange, ledit mélange comprenant une proportion de glycérol d'au moins 5 % en équivalent saccharose, la somme des proportions de glycérol et de sucre dudit mélange étant égale à 100 % en équivalent saccharose.

Une telle utilisation présente notamment un intérêt pour la production de levures à l'échelle industrielle.

L'expression au moins une souche de *Saccharomyces* signifie que la production de levures peut être réalisée à partir de plusieurs souches de levures différentes, de même espèce ou d'espèce différente.

De préférence, la production de levures est réalisée à partir d'une seule souche de levure. Le mélange de glycérol et de sucre est tel que défini ci-dessus.

Il est décrit ici l'utilisation d'un mélange de glycérol et de sucre pour la production de levures telle que définie ci-dessus, caractérisée en ce que la production de levures est réalisée selon un mode semi-continu ou continu.

Il est également décrit ici l'utilisation d'un mélange de glycérol et de sucre pour la production de levures telle que définie ci-dessus, caractérisée en ce que la production de levures est réalisée en condition aérobie.

Il est aussi décrit ici l'utilisation d'un mélange de glycérol et de sucre pour la production de levures telle que définie ci-dessus, caractérisée en ce que la production de levures est réalisée sur une durée de préférence inférieure à 48h, plus préférentiellement inférieure à 36h, encore plus préférentiellement inférieure à 24h.

De manière avantageuse, la durée de la production est comprise de 10h à 24h.

La durée de la production est par exemple de 22h.

Il est décrit ici l'utilisation d'un mélange de glycérol et de sucre pour la production de levures telle que définie ci-dessus, caractérisée en ce que le rendement de production est similaire à celui obtenu avec une souche de levure standard produite en l'absence de glycérol.

Par exemple, le rendement de production de levures est supérieur à 85 %, de préférence supérieur à 90 %, plus préférentiellement supérieur à 95 % du rendement d'une souche standard produite en l'absence de glycérol.

Par souche standard, on désigne par exemple la levure de boulangerie déposée à la NCYC sous le numéro NCYC 995.

Par « rendement » ou « rendement de production » ou « rendement de production de biomasse », on désigne le rapport de la masse de levure produite sur la masse d'équivalent saccharose mis en oeuvre.

La masse d'équivalent saccharose mise en oeuvre est constituée par la masse d'équivalent saccharose consommée et celle non consommée.

Le milieu de culture est tel que défini ci-dessus.

Selon un autre mode de réalisation, il est décrit ici l'utilisation d'un mélange de glycérol et de sucre pour la production de levures telle que définie ci-dessus, caractérisée en ce que le milieu de culture comprend une autre source de carbone assimilable par la souche de levure. Une autre source de carbone assimilable par la souche de levure est par exemple l'éthanol. Dans un tel mode de réalisation le mélange de glycérol et de sucre constitue au moins 70 % de la source carbonée pour la levure, de préférence au moins 80 % de la source carbonée pour la levure, plus préférentiellement au moins 90 % de la source carbonée pour la levure.

Il est décrit ici l'utilisation d'un mélange de glycérol et de sucre pour la production de levures telle que définie ci-dessus, caractérisée en ce que la totalité du milieu de culture n'est pas mise en présence de la souche de levure dès le début de la culture.

Le milieu de culture est de préférence mis en présence de la souche de levure de manière étalée dans le temps, à un débit constant ou variable, les différents constituants du milieu de culture pouvant être apportés en même temps ou séparément.

Il est décrit ici l'utilisation d'un mélange de glycérol et de sucre pour la production de levures telle que définie ci-dessus, caractérisée en ce que le mélange de glycérol et de sucre est introduit dans le milieu de culture de manière étalée dans le temps.

Il est décrit ici l'utilisation d'un mélange de glycérol et de sucre pour la production de levures telle que définie ci-dessus, caractérisée en ce que le mélange de glycérol et de sucre est introduit dans le milieu de culture séparément des autres constituants du milieu de culture.

Il est décrit ici l'utilisation d'un mélange de glycérol et de sucre pour la production de levures telle que définie ci-dessus, caractérisée en ce que le mélange de glycérol et de sucre est préparé avant son introduction dans le milieu de culture et/ou se forme dans le milieu de culture, lorsque le glycérol et le sucre sont apportés séparément.

Il est décrit ici l'utilisation d'un mélange de glycérol et de sucre pour la production de levures telle que définie ci-dessus, caractérisée en ce que la souche n'est pas mise en culture en présence de la totalité du glycérol et/ou de la totalité du sucre du mélange dès le début de la culture.

La présente invention a particulièrement pour objet l'utilisation telle que définie ci-dessus d'un mélange de glycérol et de sucre pour la production de levures à partir d'au moins une souche de *Saccharomyces* de l'invention, caractérisée en ce que ladite souche consomme au moins 85 % du glycérol du mélange, de préférence au moins 90 % du glycérol du mélange, plus préférentiellement au moins 95 % du glycérol du mélange, encore plus préférentiellement au moins 98 % du glycérol du mélange.

La présente invention a également pour objet l'utilisation telle que définie ci-dessus d'un mélange de glycérol et de sucre pour la production de levures à partir d'au moins une souche de Saccharomyces de l'invention, caractérisé en ce que ledit mélange comprend une proportion de glycérol d'au moins 8 % en équivalent saccharose, de préférence au moins 10 % en équivalent saccharose, plus préférentiellement au moins 12 % en équivalent saccharose, encore plus préférentiellement au moins 15 % en équivalent saccharose, encore plus préférentiellement au moins 17 % en équivalent saccharose, encore plus préférentiellement au moins 20 % en équivalent saccharose.

La présente invention a particulièrement pour objet l'utilisation telle que définie ci-dessus d'un mélange de glycérol et de sucre pour la production de levures à partir d'au moins une souche de Saccharomyces de l'invention, caractérisée en ce que la proportion de glycérol dans ledit mélange est comprise de 8 % à 30 % en équivalent saccharose, de préférence 10 % à 20 % en équivalent saccharose, plus préférentiellement de 12 % à 17 % en équivalent saccharose.

La présente invention a également pour objet l'utilisation telle que définie ci-dessus d'un mélange de glycérol et de sucre pour la production de levures à partir d'au moins une souche de Saccharomyces de l'invention, caractérisée en ce que ledit mélange comprend une proportion de glycérol d'au moins 30 % en équivalent saccharose, de préférence au moins 40 % en équivalent saccharose, plus préférentiellement au moins 50 % en équivalent saccharose, encore plus préférentiellement au moins 60 % en équivalent saccharose, encore plus préférentiellement au moins 70 % en équivalent saccharose, encore plus préférentiellement au moins 80 % en équivalent saccharose, encore plus préférentiellement au moins 90 % en équivalent saccharose.

La présente invention a également pour objet l'utilisation telle que définie ci-dessus d'un mélange de glycérol et de sucre pour la production de levures à partir d'au moins une souche de Saccharomyces de l'invention, caractérisée en ce que la proportion de glycérol dans ledit mélange est au plus de 95 % en équivalent saccharose, de préférence au plus 90 % en équivalent saccharose, plus préférentiellement au plus 85 % en équivalent saccharose.

La présente invention a particulièrement pour objet l'utilisation telle que définie ci-dessus d'un mélange de glycérol et de sucre pour la production de levures à partir d'au moins une souche de Saccharomyces de l'invention, caractérisée en ce que la proportion de glycérol dans ledit mélange est comprise de 30 % à 90 % en équivalent saccharose, de préférence 40 % à 80 % en équivalent saccharose, plus préférentiellement de 50 % à 70 % en équivalent saccharose.

La présente invention a pour objet l'utilisation telle que définie ci-dessus d'un mélange de glycérol et de sucre pour la production de levures à partir d'une souche à partir d'au moins une souche de Saccharomyces de l'invention, caractérisée en ce que ladite souche est choisie parmi les espèces *cerevisiae*, *boulardii*, *carlsbergensis* et *uvarum*.

Il est décrit ici les levures obtenues par l'utilisation telle que définie ci-dessus d'un mélange de glycérol et de sucre.

La présente invention a également pour objet un procédé de production de levures comprenant les étapes de :
introduction d'une souche de levure du genre *Saccharomyces* selon l'invention dans un fermenteur, et
multiplication de ladite souche en condition aérobie dans un milieu de culture comprenant un mélange de glycérol et de sucre, ledit mélange comprenant une proportion de glycérol d'au moins 5 % en équivalent saccharose, la somme des proportions de glycérol et de sucre dudit mélange étant égale à 100 % en équivalent saccharose.

Le mélange de glycérol et de sucre est tel que défini ci-dessus.

Le milieu de culture est tel que défini ci-dessus.

La présente invention a pour objet un procédé de production de levures tel que défini ci-dessus, caractérisé en ce que la multiplication de ladite souche est réalisée selon un mode semi-continu ou selon un mode continu, les modes semi-continu ou continu étant tels que définis ci-dessus.

La durée de culture du procédé de production de levures selon l'invention est de préférence inférieure à 48h, plus préférentiellement inférieure à 36h, encore plus préférentiellement inférieure à 24h.

De manière avantageuse, la durée de la culture du procédé de production de levures selon l'invention est comprise de 10h à 24h.

La durée de la production est par exemple de 22h.

Le pH est ajusté de préférence à une valeur comprise de 4 à 6,5, par exemple par apport simultané d'acides ou de bases.

La température est ajustée de préférence à une valeur comprise de 30°C à 34°C, par exemple par chauffage ou refroidissement du fermenteur.

De manière avantageuse, le procédé de production de levures selon l'invention donne des rendements similaires à ceux obtenus avec des souches de levure standard produites en l'absence de glycérol.

Par exemple, le rendement de production de levures selon l'invention est supérieur à 85 %, de préférence supérieur à 90 %, plus préférentiellement supérieur à 95 % du rendement d'une souche standard produite en l'absence de glycérol.

Par souche standard, on peut par exemple citer la levure de boulangerie déposée à la NCYC sous le numéro NCYC 995.

Par « rendement », on désigne le rapport de la masse de levure produite sur la masse d'équivalent saccharose mise en oeuvre.

La masse d'équivalent saccharose mise en oeuvre est constituée par la masse d'équivalent saccharose consommée et celle non consommée.

Les levures obtenues par culture de souches selon l'invention en présence d'un mélange de glycérol et de sucre et/ou les levures obtenues selon le procédé de production de levure de l'invention ont des qualités semblables aux levures classiquement utilisées et produites sur un substrat carboné de type sucre (en l'absence de glycérol).

Par le terme « qualité », on désigne à la fois la qualité fonctionnelle et les qualités de conservation.

Par exemple, lesdites levures ont des qualités semblables en terme de résistance au séchage, en terme de rendement de production, en terme de pouvoir fermentaire pour les levures de panification, en terme de production d'alcool et/ou de résistance à de fortes concentrations en alcool pour les levures alcool aux levures classiquement utilisées et produites sur un substrat carboné de type sucre (en l'absence de glycérol).

Les levures obtenues par culture de souches selon l'invention en présence d'un mélange de glycérol et de sucre et/ou les levures obtenues selon le procédé de production de levure de l'invention ont une concentration intracellulaire en glycérol de préférence inférieure à 1,5 % en masse de matière sèche, plus préférentiellement inférieure à 1 %.

Cette faible concentration en glycérol intracellulaire reflète l'absence d'accumulation de glycérol dans le milieu et donc le fait qu'il a été métabolisé.

Dans un mode de réalisation particulier, le milieu de culture comprend une autre source de carbone assimilable par la souche de levure. Une autre source de carbone assimilable par la souche de levure est par exemple l'éthanol. Dans un tel mode de réalisation, le mélange de glycérol et de sucre constitue au moins 70 % de la source carbonée pour la levure, de préférence au moins 80 % de la source carbonée pour la levure, plus préférentiellement au moins 90 % de la source carbonée pour la levure.

Dans un mode de réalisation préféré, la totalité du milieu de culture n'est pas mise en présence de la souche de levure dès le début de la culture. Le milieu de culture est de préférence mis en présence de la souche de levure de manière étalée dans le temps, à un débit constant ou variable, les différents constituants du milieu de culture pouvant être apportés en même temps ou séparément.

Dans un mode de réalisation préféré selon l'invention, le mélange de glycérol et de sucre est introduit dans le milieu de culture de manière étalée dans le temps.

Dans un mode de réalisation préféré selon l'invention, le mélange de glycérol et de sucre est introduit dans le milieu de culture séparément des autres constituants du milieu de culture.

Le mélange de glycérol et de sucre peut être préparé avant son introduction dans le milieu de culture et/ou se former dans le milieu de culture, lorsque le glycérol et le sucre sont apportés séparément.

Dans un mode de réalisation préféré selon l'invention, la souche n'est pas mise en culture en présence de la totalité du glycérol et/ou de la totalité du sucre du mélange dès le début de la culture.

La présente invention a également pour objet un procédé de production de levure tel que défini ci-dessus comprenant les étapes de :
- introduction d'une souche de levure du genre *Saccharomyces* selon l'invention dans un fermenteur,
- éventuellement multiplication de ladite souche en condition aérobie dans un milieu de culture ne comprenant pas de glycérol,
- multiplication de ladite souche en condition aérobie, selon un mode semi-continu ou continu, dans un milieu de culture comprenant un mélange de glycérol et de sucre, ledit mélange comprenant une proportion de glycérol d'au moins 5 % en équivalent saccharose, la somme des proportions de glycérol et de sucre dudit mélange étant égale à 100 % en équivalent saccharose,
- éventuellement multiplication de ladite souche en condition aérobie dans un milieu de culture ne comprenant pas de glycérol.

La présente invention a pour objet un procédé de production de levures comprenant les étapes de :
introduction d'une souche de levure du genre *Saccharomyces* selon l'invention dans un fermenteur,
éventuellement multiplication de ladite souche en condition aérobie dans un milieu de culture ne comprenant pas de glycérol,
multiplication de ladite souche en condition aérobie dans un milieu de culture comprenant un mélange de glycérol et de sucre, ledit mélange comprenant une proportion de glycérol d'au moins 5 % en équivalent saccharose, la somme des proportions de glycérol et de sucre dudit mélange étant égale à 100 % en équivalent saccharose,
éventuellement, multiplication de ladite souche en condition aérobie dans un milieu de culture ne comprenant pas de glycérol, et
séparation par centrifugation de la levure de boulangerie ainsi produite de son milieu de culture, avec l'obtention d'une « crème de levure » liquide contenant environ entre 14 et 25 % de matières sèches, voire des matières sèches plus élevées si la crème de levure est mélangée avec des produits osmotiques.

La présente invention a également pour objet le procédé de production de levures telle que défini ci-dessus comprenant des étapes ultérieures éventuelles de :
- filtration de la crème de levure liquide ainsi obtenue, en général sur un filtre rotatif sous vide, et l'obtention d'une levure fraîche déshydratée contenant environ 26 à 35 % de matières sèches,
- malaxage de ladite levure fraîche déshydratée en vue de l'obtention d'une masse bien homogène,
- extrusion de la levure ainsi obtenue et obtention d'une levure pressée sous formes de pains de levure fraîche ou de levure fraîche émiettée, commercialisés à environ 30 % de matières sèches, ou si la levure est destinée à être séchée, sous forme de vermicelles,
- séchage de manière ménagée, dans un courant d'air chaud, par exemple par fluidisation, des particules de levures obtenues par extrusion, et
- emballage.

La présente invention a particulièrement pour objet un procédé de production de levures tel que défini ci-dessus, caractérisé en ce qu'au moins 80 % du glycérol dudit mélange est consommé par ladite souche selon l'invention, de préférence au moins 85 % du glycérol dudit mélange, plus préférentiellement au moins 90 % du glycérol dudit mélange, encore plus préférentiellement au moins 95 % du glycérol dudit mélange, encore plus préférentiellement au moins 98 % du glycérol dudit mélange.

La présente invention a également pour objet un procédé de production de levures tel que défini ci-dessus, caractérisé en ce que ledit mélange comprend une proportion de glycérol d'au moins 8 % en équivalent saccharose, de préférence au moins 10 % en équivalent saccharose, plus préférentiellement au moins 12 % en équivalent saccharose, encore plus préférentiellement au moins 15 % en équivalent saccharose, encore plus préférentiellement au moins 17 % en équivalent saccharose, encore plus préférentiellement au moins 20 % en équivalent saccharose.

La présente invention a particulièrement pour objet un procédé de production de levures tel que défini ci-dessus, caractérisé en ce que la proportion de glycérol dans ledit mélange est comprise de 8 % à 30 % en équivalent saccharose, de préférence 10 % à 20 % en équivalent saccharose, plus préférentiellement de 12 % à 17 % en équivalent saccharose.

La présente invention a particulièrement pour objet un procédé de production de levures tel que défini ci-dessus, caractérisé en ce que ledit mélange comprend une proportion de glycérol d'au moins 30 % en équivalent saccharose.

La présente invention a plus particulièrement pour objet un procédé de production de levures tel que défini ci-dessus, caractérisé en ce que ledit mélange comprend une proportion de glycérol d'au moins 40 % en équivalent saccharose, de préférence au moins 50 % en équivalent saccharose, plus préférentiellement au moins 60 % en équivalent saccharose, encore plus préférentiellement au moins 70 % en équivalent saccharose, encore plus préférentiellement au moins 80 % en équivalent saccharose, encore plus préférentiellement au moins 90 % en équivalent saccharose.

La présente invention a également pour objet un procédé de production de levures tel que défini ci-dessus, caractérisé en ce que la proportion de glycérol dans ledit mélange est au plus de 95 % en équivalent saccharose, de préférence au plus 90 % en équivalent saccharose, plus préférentiellement au plus 85 % en équivalent saccharose.

La présente invention a particulièrement pour objet un procédé de production de levures tel que défini ci-dessus, caractérisé en ce que la proportion de glycérol dans ledit mélange est comprise de 30 % à 90 % en équivalent saccharose, de préférence 40 % à 80 % en équivalent saccharose, plus préférentiellement de 50 % à 70 % en équivalent saccharose.

La présente invention a pour objet un procédé de production de levures tel que défini ci-dessus, caractérisé en ce que ladite souche de levure selon l'invention est choisie parmi les espèces *cerevisiae*, *boulardii*, *carlsbergensis* et *uvarum*.

Il est décrit ici un procédé de production de levures tel que défini ci-dessus, caractérisé en ce que ladite souche de levure est choisie parmi un variant naturel et/ou une souche génétiquement modifiée.

Il est décrit ici un procédé de production de levures tel que défini ci-dessus, caractérisé en ce que ladite souche de levure est choisie parmi la souche déposée le 20 décembre 2007 auprès de la CNCM sous le numéro CNCM I-3886, la souche déposée le 20 décembre 2007 auprès de la CNCM sous le numéro CNCM I-3887, la souche déposée le 20 décembre 2007 auprès de la CNCM sous le numéro CNCM I-3888, la souche déposée le 3 mars 2009 auprès de la CNCM sous le numéro I-4132, la souche déposée le 3 mars 2009 auprès de la CNCM sous le numéro I-4133, la souche déposée le 3 mars 2009 auprès de la CNCM sous le numéro I-4129, la souche déposée le 3 mars 2009 auprès de la CNCM sous le numéro I-4130 ou la souche déposée le 3 mars 2009 auprès de la CNCM sous le numéro I-4131, ou leur mélange.

La présente invention a également pour objet des levures susceptibles d'être obtenues par la mise en oeuvre du procédé de production de levures tel que défini ci-dessus.

Dans un mode de réalisation particulier de l'invention, le glycérol résiduel est recyclé afin d'être utilisé dans un mélange de glycérol et de sucre.

Selon un autre mode de réalisation, le mélange constitué de glycérol et de sucre peut comprendre une proportion de glycérol allant jusqu'à 99,9 % en équivalent saccharose, voire quasiment 100 % en équivalent saccharose.

Les exemples suivants illustrent l'invention sans la limiter.

### EXEMPLES

### EXEMPLE 1 : Obtention de souches de levure génétiquement modifiées selon l'invention

### Matériel et Méthodes

Le gène STL1 (YDR536w) code la perméase active au glycérol. Ce gène a été amplifié à partir de l'ADN génomique d'une levure industrielle et séquencé.

La séquence du gène STL1 amplifié, composé de 1710 nucléotides, a été alignée sur la séquence nucléotidique de l'ORF YDR536w issu de la levure S288c (banque SGD). L'alignement a mis en évidence la présence de 7 nucléotides différents sur toute la séquence, ce qui correspond à un pourcentage d'identité de 99,6 %.

La traduction de la séquence nucléotidique du gène STL1 issu de la levure industrielle, conduit à une protéine de 570 acides aminés. L'alignement de la séquence protéique du gène STL1 issu de la levure industrielle avec la séquence protéique de l'ORF YDR536w issu de la levure S288c a mis en évidence la présence de 3 acides aminés différents, ce qui correspond à un pourcentage d'identité de 99.5 %.

La construction de souches de levure industrielles génétiquement modifiées repose sur un outil moléculaire adapté aux levures industrielles construit par la Demanderesse.

Cet outil permet notamment :
le clonage d'un gène d'intérêt, ici le gène STL1, entre le promoteur ADHI et le terminateur CYC1 de levure, ce qui permet l'expression de ce gène dans la levure,
la présence de part et d'autre de la cassette d'expression de 2 fragments recombinogéniques (BUD5-A et BUD5-B, de 500 nucléotides chacun), ce qui permet l'insertion par recombinaison homologue de la cassette dans le génome de la levure, après transformation, au niveau du locus BUD5 (YCR038c) situé sur le chromosome III,
la présence du marqueur de sélection KANMX, encadré de 2 sites loxP, ce qui permet la sélection de clones de levures ayant intégré dans leur génome la cassette d'expression contenant le gène STL1 ; ces mêmes clones sont ensuite transformés avec un plasmide portant le gène Cre codant pour une recombinase placée sous le contrôle du promoteur Gall ; après induction de l'expression de ce gène par culture en milieu contenant du galactose, l'action de la recombinase va induire la perte du fragment d'ADN contenant le marqueur de sélection KANMX.

La cassette d'expression est représentée à la figure 1.

Les 5 souches de départ utilisées sont les suivantes :
- la souche de *Saccharomyces cerevisiae* déposée à la CNCM sous le numéro I-3399,
- la souche de *Saccharomyces cerevisiae* déposée à la NCYC sous le numéro NCYC996,
- la souche de *Saccharomyces cerevisiae* déposée à la NCYC sous le numéro NCYC995,
- la souche de *Saccharomyces cerevisiae* déposée à la CNCM sous le numéro I-4072, et
- la souche de *Saccharomyces cerevisiae* déposée à la CNCM sous le numéro I-4071.

Chacune des souches de départ est transformée avec la cassette d'expression. Les clones résistants à la généticine sont sélectionnés. Ensuite, pour chaque clone sélectionné, l'intégration de la cassette d'expression dans le génome de la levure au bon locus est vérifiée par PCR. Les clones sélectionnés sont ensuite transformés avec un plasmide portant le gène Cre codant pour une recombinase placée sous le contrôle du promoteur Gall. Après induction de l'expression de ce gène Cre par culture en milieu contenant du galactose, l'action de la recombinase permet d'induire la perte du fragment d'ADN contenant le marqueur de sélection KANMX. On vérifie alors la perte dudit marqueur de sélection KANMX au niveau du site d'intégration dans le chromosome par PCR, la présence de la cassette d'expression contenant le gène STL1 dans le génome de la levure au bon locus par PCR, ainsi que la perte de la résistance à la généticine. La perte du plasmide Cre est vérifiée par la perte de la résistance à la nourséothrycine.

Enfin, la transcription du gène STL1 en présence de sucre dans la souche génétiquement modifiée est vérifiée en mesurant le taux d'ARNm issu de la transcription du gène STL1 placé sous le contrôle du promoteur ADH1. A cet effet, la souche modifiée est cultivée en présence de sucre (milieu de culture de laboratoire YPG contenant 2 % de glucose). L'ARNm est extrait desdites souches et le taux d'ARNm correspondant à la transcription du gène STL1 est mesuré par PCR quantitative. Le taux d'ARNm de la souche mutée est alors exprimé en fonction du taux d'ARNm de la souche non mutée correspondante (fixée à 1).

### Résultats

Les souches génétiquement modifiées pour exprimer le gène STL1 ainsi obtenues ont été déposées à la CNCM sous les numéros suivants :
- la souche numéro I-3886, issue de la souche de départ déposée à la CNCM sous le numéro I-3399,
- la souche numéro I-3887, issue de la souche de départ déposée à la NCYC sous le numéro NCYC996,
- la souche numéro I-3888, issue de la souche de *Saccharomyces cerevisiae* de départ déposée à la NCYC sous le numéro NCYC995,
- la souche numéro I-4132, issue de la souche de départ déposée à la CNCM sous le numéro I-4072, et
- la souche numéro I-4133, issue de la souche de départ déposée à la CNCM sous le numéro I-4071.

Les taux de transcription indiqués dans la figure 2 montrent une forte augmentation (de 270 à 300 fois) de la transcription du gène STL1 chez les trois souches génétiquement modifiées I-3886, I-3887 et I-3888 cultivées en présence de sucre, en comparaison avec le niveau de transcription du gène dans la souche témoin non mutée correspondante cultivée dans le même milieu.

S'agissant du taux de transcription des souches I-4132 et I-4133 cultivées en présence de sucre, une forte augmentation (supérieure à 200 fois) de la transcription du gène STL1 est également mise en évidence, en comparaison avec le niveau de transcription du gène dans la souche témoin non mutée correspondante cultivée dans le même milieu.

### EXEMPLE 2 : Production de levures à partir de souches génétiquement modifiées sur un mélange de glycérol et de sucre

### Matériel et Méthodes

### (i) Mode semi-continu

La production de levure fraîche est menée de la manière décrite dans l'ouvrage « Yeast technology» », 2ème édition, 1991, G. Reed et T.W. Nagodawithana, publié par Van Nostrand Reinhold, ISBN 0-442-31892-8.

Le fermenteur utilisé est ensemencé avec un certain volume initial contenant de l'eau, un peu d'azote et de phosphore, des éléments nutritifs comme des vitamines et des minéraux.

Avant d'ensemencer le fermenteur avec une certaine quantité de souches de levure vivantes, un peu de mélasse est ajouté pour démarrer la culture. Le pH et la température sont ajustés à des valeurs comprises respectivement de 3,5 à 7 pour le pH (avec des apports simultanés d'acide ou de base selon les besoins), et de 30 à 34°C pour la température (avec un chauffage ou un refroidissement du fermenteur selon les besoins). La culture en mode semi-continue dite fed-batch est démarrée par apport de mélasse ou d'un mélange de mélasse et de glycérol comprenant une proportion de glycérol de 20 % en équivalent saccharose. La vitesse de coulée de la mélasse ou du mélange de glycérol et de mélasse détermine la vitesse de croissance des levures. Cette vitesse de coulée variable est fixée au cours du temps de manière à ce que l'équivalent saccharose apporté au cours du temps puisse être totalement respiré (oxydé) par la levure. Dans de telles conditions, la production d'éthanol par la levure au cours du temps est nulle, ou limitée aux premières heures de l'essai. Afin de respecter ces conditions, la culture est par exemple réalisée à un taux de croissance moyen de 0,16 h-1 pour les souches I-3886, 1-3887,1-3888 et les souches non OGM correspondantes, et à un taux de croissance moyen de 0,13 h-1 pour les souches I-4132 et I-4133 et les souches non OGM correspondantes.

Le milieu est suffisamment aéré et mélangé pour ne pas être limitant en oxygène.

En complément de la mélasse ou du mélange de glycérol et de mélasse, une source continue d'azote sous la forme d'urée et de phosphore sous la forme d'acide phosphorique permet de répondre aux besoins de la levure pour la synthèse des protéines et des autres constituants cellulaires.

L'apport de mélasse ou du mélange de glycérol et de mélasse est maintenu pendant 22 heures de manière à obtenir la quantité de levure souhaitée, compte tenu des équipements mis en oeuvre.

Au cours des dernières heures de culture, l'apport de mélasse ou du mélange de glycérol et de mélasse est diminué progressivement de manière à réduire le taux de levures bourgeonnantes dans le fermenteur.

Selon ce procédé de mise en oeuvre, l'alimentation en substrat carboné (par exemple le mélange de glycérol et de mélasse comprenant une proportion de glycérol de 20 % en équivalent sucre) permet de multiplier la biomasse initiale par 40 en 22h de culture, tout en maintenant un métabolisme majoritairement respiratoire et en garantissant un taux de bourgeon à la récolte inférieur à 5-10 %. La quantité totale de substrat carboné coulée est fonction de la capacité de transfert en oxygène et calorie de la cuve de fermentation.

La concentration en biomasse finale est comprise entre 5 et 7 % et la teneur en azote de cette biomasse entre 6 et 9 %.

Au cours de la culture et en fin de culture, la quantité de glycérol dans le milieu de culture est mesurée par HPLC et est comparée à la quantité de glycérol apporté.

A la fin de la culture, la levure est récoltée par centrifugation et la masse de levure récoltée est mesurée.

La levure produite (en g de MS) est la différence entre la levure récoltée (en g de MS) et la levure de l'inoculum (en g de MS) (MS signifiant Matière Sèche).

Le rendement en pourcentage est alors obtenu en faisant le rapport « levure produite (g de MS) » / « substrat carboné introduit en équivalent saccharose (g) » et en multipliant par 100.

Le pourcentage de glycérol consommé est calculé en faisant le rapport « quantité de glycérol mesurée en fin de culture (g) » / « quantité de glycérol apportée au cours de la culture (g) » et en multipliant par 100.

### (ii) Mode chemostat

Le fermenteur est alimenté par une source de carbone constituée par un mélange de glycérol et de sucre, une source d'azote, une source de soufre et une source de phosphore. Des vitamines et minéraux sont apportés en excès.

Des mélanges de glycérol et de glucose comportant de 0 % à 80 % de glycérol en équivalent saccharose sont testés.

Le fermenteur est placé sur une balance qui contrôle une pompe de soutirage afin de maintenir le volume dans le fermenteur constant par la maîtrise de son poids. La culture est limitée par le substrat carboné (glucose et glycérol), les autres constituants étant apportés en excès. Le milieu est suffisamment aéré et mélangé pour ne jamais être limitant en oxygène.

Le milieu est apporté en continu avec un débit constant permettant d'obtenir un taux de dilution (=Débit/volume) de 0.15 h⁻¹ et donc en régime permanent un taux de croissance constant de 0.15 h⁻¹.

La concentration en équivalent saccharose dans le milieu d'alimentation est égale à 19 g/kg.

Le rendement ainsi que la quantité résiduelle de glycérol sont mesurés sur la culture en régime permanent.

Le régime permanent est atteint lorsque les concentrations dans le fermenteur sont stables pendant au moins 3 temps de séjour, le temps de séjour étant le rapport 1/[taux de dilution].

Le rendement de production de biomasse sur sucre coulé est le rapport de la biomasse produite sur le sucre coulé. En régime permanent, la biomasse produite par unité de temps est égale au débit de soutirage multiplié par la concentration de biomasse dans le fermenteur (déterminée en filtrant un volume connu de mout levuré et en pesant le gâteau de levure préalablement lavé et séché).

Au moment de la détermination du rendement un échantillon de mout levuré est prélevé et immédiatement filtré pour éliminer la biomasse. Le filtrat est analysé par HPLC pour doser la quantité résiduelle de glycérol.

### Résultats

### (i) Mode semi-continu

Les souches non mutées I-3399, NCYC 996, NCYC 995, I-4072 et I-4071 et les souches correspondantes mutées, I-3886, I-3887, I-3888, I-4132 et I-4133 respectivement, ont été produites selon le schéma de mise en oeuvre décrit ci-dessus, avec comme source de carbone de la mélasse pure ou un mélange de glycérol et de mélasse comprenant une proportion de glycérol de 20 % en équivalent saccharose.

Les figures 3 et 4 et les tableaux 1, 2, 3, 4 et 5 illustrent les résultats obtenus avec les souches non mutées et les souches correspondantes mutées.

Le rendement de production est déterminé pour chaque souche non mutée en présence de mélasse pure (sans glycérol). Dans les tableaux 1, 2, 3, 4 et 5, les valeurs indiquées à la ligne « rendement » correspondent au rendement en pourcentage par rapport au rendement de la souche non mutée produite sur mélasse pure.

Le tableau 1 indique les résultats des essais réalisés avec la souche NCYC 996 non mutée et la souche correspondante mutée I-3887.

Le tableau 2 indique les résultats des essais réalisés avec la souche I-3399 non mutée et la souche correspondante mutée I-3886.

Le tableau 3 indique les résultats des essais réalisés avec la souche NCYC 995 non mutée et la souche correspondante mutée I-3888.

Le tableau 4 indique les résultats des essais réalisés avec la souche I-4072 non mutée et la souche correspondante mutée I-4132.

Le tableau 5 indique les résultats des essais réalisés avec la souche I-4071 non mutée et la souche correspondante mutée I-4133.

A la lecture de ces tableaux, on constate que lors d'une production sur un mélange de glycérol et de mélasse (comprenant une proportion de glycérol de 20 % en équivalent saccharose) comme source de carbone, le rendement de production obtenu avec les souches non mutées est significativement inférieur au rendement de production obtenu sur mélasse pure. Cette diminution du rendement est à relier à la non utilisation d'une partie significative de l'équivalent saccharose apporté sous la forme de glycérol, le glycérol s'accumulant dans le milieu de culture comme indiqué dans les résultats à la ligne « glycérol apporté consommé ».

Dans ces mêmes tableaux sont indiqués les résultats des essais réalisés avec les souches mutées, lorsque la source de carbone apportée est un mélange de mélasse et de glycérol. Les mesures effectuées indiquent que la consommation du glycérol apporté est pratiquement totale pour les trois souches mutées I-3887, I-3886 et I-3888 (consommation de glycérol supérieure à 99 %), ce qui se traduit par des rendements de production au moins équivalents à ceux obtenus avec des souches non mutées produites sur mélasse pure (tableaux 1 à 3).

S'agissant des souches mutées I-4132 et I-4133, la consommation du glycérol apporté est supérieure à 80 % et le rendement est supérieur à 90 % du rendement obtenu avec la souche non OGM en l'absence de glycérol (tableaux 4 et 5).

Cette différence de consommation du glycérol par les souches non mutées et les souches mutées correspondantes est illustrée dans les figures 3 et 4 qui présentent l'accumulation de glycérol au cours du temps, lors des essais de production sur un mélange de glycérol et de mélasse comprenant une proportion de 20 % de glycérol en équivalent saccharose.

La courbe en triangle de la figure 3 représente la quantité résiduelle de glycérol dans le milieu débarrassé des levures au cours de la culture de la souche non mutée NCYC 996 et la courbe en carré celle de la souche mutée I-3887.

La courbe en triangle de la figure 4 représente la quantité résiduelle de glycérol dans le milieu débarrassé des levures au cours de la culture de la souche non mutée NCYC 995 et la courbe en carré celle de la souche mutée I-3888.

La différence de consommation entre les souches non mutées et les souches mutées correspondantes sont significatives après les cinq premières heures de culture. Au cours des cinq premières heures de culture, la capacité respiratoire des souches non mutées et des souches mutées est saturée par le sucre apporté (effet « crabtree »), ce phénomène s'accompagnant d'une production d'éthanol. Lorsque ce phénomène disparaît, c'est-à-dire lorsque les levures ont la capacité respiratoire d'oxyder la totalité du sucre et du glycérol apporté, les souches mutées consomment la quasi-totalité du glycérol coulé simultanément au sucre, alors que les souches non mutées accumulent le glycérol.

**Tableau 1**

| | 0 % glycérol | 20 % glycérol | |
|---|---|---|---|
| | NCYC 996 | NCYC 996 | I-3887 |
| Rendement (en pourcentage du rendement de la souche témoin en l'absence de glycérol) | 100,0 | 89,6 | 102,5 |
| Glycérol apporté consommé (en %) | / | 65,5 | 99,2 |

**Tableau 2**

| | 0 % glycérol | 20 % glycérol | |
|---|---|---|---|
| | I-3399 | I-3399 | I-3886 |
| Rendement (en pourcentage du rendement de la souche témoin en l'absence de glycérol) | 100,0 | 93,8 | 108,1 |
| Glycérol apporté consommé (en %) | / | 48,9 | 99,1 |

**Tableau 3**

| | 0 % glycérol | 20 % glycérol | |
|---|---|---|---|
| | NCYC 995 | NCYC 995 | I-3888 |
| Rendement (en pourcentage du rendement de la souche témoin en l'absence de glycérol) | 100,0 | 93,7 | 100,9 |
| Glycérol apporté consommé (en %) | / | 57,9 | 98,9 |

**Tableau 4**

| | 0 % glycérol | 20 % glycérol | |
|---|---|---|---|
| | I-4072 | I-4072 | I-4132 |
| Rendement (en pourcentage du rendement de la souche témoin en l'absence de glycérol) | 100,0 | 78,7 | 93,3 |
| Glycérol apporté consommé (en %) | / | 22,7 | 83,9 |

**Tableau 5**

| | 0 % glycérol | 20 % glycérol | |
|---|---|---|---|
| | I-4071 | I-4071 | I-4133 |
| Rendement (en pourcentage du rendement de la souche témoin en l'absence de glycérol) | 100,0 | 88,2 | 92,9 |
| Glycérol apporté consommé (en %) | / | 44,8 | 84,2 |

### (ii) Mode continu

Dans les figures 5 et 6, chaque point correspond à un régime permanent obtenu avec un milieu d'alimentation dans lequel la proportion de glycérol dans le mélange de glycérol et de sucre est de 0 %, 20 %, 50 % ou 80 % en équivalent sucre.

Avec un milieu d'alimentation dont la seule source de carbone est le glucose (absence de glycérol), le rendement de production de biomasse de la souche mutée (CNCM I-3887) et celui de la souche non mutée (NCYC 996) sont identiques. La mutation du gène STL1 ne modifie donc pas le rendement de production de biomasse sur glucose.

La substitution de 20 %, 50 % ou 80 % du glucose en équivalent saccharose par du glycérol dans le mélange de glycérol et de sucre de l'alimentation s'accompagne, pour la souche non mutée, d'une accumulation croissante de glycérol dans le milieu réactionnel (figures 7 et 8). Cette quantité de glycérol qui s'accumule dans le milieu réactionnel entraîne une diminution prononcée du rendement de production de biomasse sur équivalent sucre coulé (figures 5 et 6). Ainsi, à un taux de 50 % de substitution, le rendement de production des souches non mutées a chuté de plus de 25 % par rapport au rendement de la même souche produite sur un milieu ne contenant que du glucose.

La consommation de glycérol des souches non mutées est inférieure à 57 % à un taux de substitution de 20 %, inférieure à 38 % à un taux de substitution de 50 % et inférieure à 22 % à un taux de substitution de 80 % (figures 7 et 8).

Au contraire, les souches mutées consomment la quasi totalité du glycérol coulé, simultanément au sucre, comme le montre la très faible concentration en glycérol résiduel mesurée dans le milieu réactionnel. Ainsi, les souches mutées consomment plus de 95 % du glycérol du mélange, et ce jusqu'à un taux de substitution de 80 % (figures 7 et 8). En conséquence, le rendement de production sur équivalent sucre coulé n'est pas ou est peu affecté par le taux de substitution de sucre par du glycérol. Le rendement de production de la souche mutée est toujours supérieur à celui de la souche non mutée correspondante en présence d'un mélange contenant de glycérol (figures 5 et 6).

Par ailleurs, en présence d'un mélange de glycérol et de sucre comprenant 20 % ou 50 % de glycérol en équivalent saccharose, le rendement de production des souches mutées est supérieur à 90 % par rapport à celui de la souche non muté cultivée en l'absence de glycérol, et il est supérieur à 85 % en présence d'un mélange de glycérol et de sucre comprenant 80 % de glycérol en équivalent saccharose (figures 5 et 6).

### EXEMPLE 3 : Obtention de variants naturels

### Matériel et Méthodes

### (i) Criblage direct

Une population de cellules d'une souche de levure de panification standard est soumise à un rayonnement UV de 1200 à 2000 J/cm² conduisant à une perte de viabilité de 99,9 %.

Les variants naturels obtenus sont isolés sur un milieu non sélectif (YPG), puis sont criblés sur la base de leur rendement et de leur consommation en glycérol en présence d'un mélange de glycérol et de sucre.

Le criblage est effectué après culture en mode semi-continu, en présence d'un mélange de glycérol et de sucre comprenant 20 % de glycérol en équivalent saccharose, dans des fermenteurs de 1 litre, puis de 7 litres.

Les conditions expérimentales de la culture semi-continue sont telles que décrites dans l'exemple 2 sur une durée de 22h, avec un taux de croissance moyen de 0,16 h⁻¹.

### (ii) Criblage après enrichissement en chemostat

Une population de cellules de la même souche de départ que précédemment est soumise à des taux croissants de rayonnement UV (de 400 à 4400 J/cm²) conduisant à des pertes de viabilité allant de 80 à 99,98 %.

Les variants naturels soumis à ces différents taux de rayonnement sont ensuite regroupés et une partie est utilisée pour ensemencer une culture en mode chemostat.

La source de carbone de la culture est constituée par un mélange de glycérol et de glucose comprenant 50 % de glycérol en équivalent saccharose.

La culture est conduite jusqu'à obtenir environ 220 générations cellulaires.

La consommation en glycérol est mesurée au cours de la culture.

Des prélèvements de la culture sont effectués régulièrement en vue du criblage des variants naturels.

Les variants naturels issus des différents prélèvements sont isolés sur un milieu non sélectif (YPG), puis sont criblés sur la base de leur rendement et de leur consommation en glycérol en présence d'un mélange de glycérol et de sucre.

Le criblage est effectué après culture en mode semi-continu, en présence d'un mélange de glycérol et de sucre comprenant 20 % de glycérol en équivalent saccharose, dans des fermenteurs de 1 litre, puis de 7 litres.

Les conditions expérimentales de la culture semi-continue sont telles que décrites dans l'exemple 2 sur une durée de 22h, avec un taux de croissance moyen de 0,16.

### Résultats

Les variants naturels issus du criblage direct ou du criblage après enrichissement en chemostat présentent des profils de consommation du glycérol et de rendement très variés.

Un exemple de résultat obtenu sur 53 variants naturels issus du criblage après enrichissement en chemostat est présenté en figure 9. La consommation en glycérol de la souche de départ (T) est également indiquée.

Un certain nombre de variants naturels présente une consommation de glycérol supérieure à 80 % en présence d'un mélange de glycérol et de sucre comprenant 20 % de glycérol en équivalent saccharose et un rendement supérieur ou égal à 85 % de la souche de départ.

Parmi ceux-ci figurent les variants naturels déposés à la CNCM sous les numéros I-4129 (variant naturel n° 4), I-4130 (variant naturel n° 20) et I-4131 (variant naturel n° 2) qui ont été obtenus par criblage après enrichissement en chemostat à partir du prélèvement correspondant à l'obtention d'environ 145 générations cellulaires dans le chemostat.

On note que l'analyse de la consommation en glycérol au cours de la culture en chemostat montre qu'au cours des 100 premières générations, le glycérol résiduel diminue régulièrement, ce qui suggère une adaptation de la culture au mélange de glycérol et de sucre.

Par ailleurs, le taux d'ARNm correspondant à la transcription du gène STL1 est mesuré par RT-PCR quantitative, comme indiqué dans l'exemple 1, sur différents variants naturels. Le taux d'ARNm obtenu est alors comparé à celui de la souche de départ n'ayant pas subi de traitement UV.

Selon les variants naturels, on observe un taux d'ARNm similaire à celui de la souche de départ ou multiplié d'un facteur 2 à un facteur 7.

La capacité des souches à consommer du glycérol en présence d'un mélange de glycérol et de sucre n'est donc pas nécessairement liée à une surexpression du gène STL1.

### EXEMPLE 4 : Production de levures à partir de variants naturels sur un mélange de glycérol et de sucre

### Matériel et Méthodes

Les cultures en mode semi-continu sont réalisées comme dans l'exemple 2, sur une durée de 22h, avec taux de croissance moyen de 0,16 h⁻¹.

### Résultats

Les variants naturels I-4129, I-4130, et I-4131, ainsi que la souche de levure de départ dont ils sont issus sont produits selon le schéma de mise en oeuvre décrit ci-dessus, avec comme source de carbone de la mélasse pure ou un mélange de glycérol et de mélasse comprenant une proportion de glycérol de 20 % ou 40 % en équivalent saccharose.

Le rendement de production est déterminé pour la souche de départ en présence de mélasse pure (sans glycérol). Dans les tableaux 6 et 7, les valeurs indiquées à la ligne « rendement » correspondent au rendement en pourcentage par rapport au rendement de la souche de départ produite sur mélasse pure.

Les tableaux 6 et 7 indiquent les résultats des essais réalisés avec les variants naturels et la souche de départ (T) en présence d'un mélange de glycérol et de sucre comprenant respectivement 20 % et 40 % de glycérol.

**Tableau 6**

| | 0 % glycérol | 20 % glycérol | | | |
|---|---|---|---|---|---|
| | T | T | I-4129 | I-4130 | I-4131 |
| Rendement (en pourcentage du rendement de la souche témoin en l'absence de glycérol) | 100,0 | 86,5 | 98,9 | 100,3 | 100,6 |
| Glycérol apporté consommé (en %) | / | 38,5 | 99,6 | 99,6 | 99,5 |

**Tableau 7**

| | 0 % glycérol | 40 % glycérol | | |
|---|---|---|---|---|
| | T | I-4129 | I-4130 | I-4131 |
| Rendement (en pourcentage du rendement de la souche témoin en l'absence de glycérol) | 100,0 | 99,7 | 88,2 | 93,5 |
| Glycérol apporté consommé (en %) | / | 99,1 | 85,6 | 93,1 |

Les mesures effectuées indiquent que la consommation du glycérol apporté est pratiquement totale pour les trois variants naturels en présence d'un mélange de glycérol et de sucre comprenant 20 % de glycérol en équivalent saccharose (consommation de glycérol supérieure à 99 %), alors que la consommation de la souche de départ est inférieure à 40 % (tableau 6).

En présence d'un mélange de glycérol et de sucre comprenant 40 % de glycérol en équivalent saccharose, les mesures effectuées indiquent que la consommation du glycérol apporté est pratiquement totale pour le variant naturel I-4129, supérieure à 85 % pour le variant naturel I-4130 et supérieure à 90 % pour le variant I-4131.

## Revendications

1. Souche de *Saccharomyces*, **caractérisée en ce qu'**en présence d'un milieu de culture comprenant un mélange de glycérol et de sucre, en condition aérobie, elle consomme au moins 80% du glycérol dudit mélange tout en consommant du sucre simultanément, ledit mélange comprenant une proportion de glycérol d'au moins 20% en équivalent saccharose, la somme des proportions de glycérol et de sucre dudit mélange étant égale à 100% en équivalent saccharose, la souche de *Saccharomyces* étant une souche modifiée génétiquement pour sur-exprimer le gène STL1 en présence de sucre ou étant choisie parmi la souche déposée le 3 mars 2009 auprès de la CNCM sous le numéro CNCM I-4129, la souche déposée le 3 mars 2009 auprès de la CNCM sous le numéro CNCM I-4130 et la souche déposée le 3 mars 2009 auprès de la CNCM sous le numéro CNCM I-4131.

2. Souche selon la revendication 1, **caractérisée en ce qu'**elle consomme au moins 85% du glycérol du mélange, de préférence au moins 90% du glycérol du mélange, plus préférentiellement au moins 95% du glycérol du mélange, encore plus préférentiellement au moins 98% du glycérol du mélange.

3. Souche selon la revendication 1 ou la revendication 2, **caractérisée en ce que** ledit mélange comprend une proportion de glycérol d'au moins 30% en équivalent saccharose, de préférence au moins 40% en équivalent saccharose, plus préférentiellement au moins 50% en équivalent saccharose, encore plus préférentiellement au moins 60% en équivalent saccharose, encore plus préférentiellement au moins 70% en équivalent saccharose, encore plus préférentiellement au moins 80% en équivalent saccharose, encore plus préférentiellement au moins 90% en équivalent saccharose.

4. Souche selon l'une des revendications 1 à 3, **caractérisée en ce qu'**elle est choisie parmi les espèces *cerevisiae*, *boulardii, carlsbergensis* et *uvarum*.

5. Souche selon l'une quelconque des revendications 1 à 4, **caractérisée en ce qu'**elle est choisie parmi la souche déposée le 20 décembre 2007 auprès de la CNCM sous le numéro CNCM I-3886, la souche déposée le 20 décembre 2007 auprès de la CNCM sous le numéro CNCM I-3887, la souche déposée le 20 décembre 2007 auprès de la CNCM sous le numéro CNCM I-3888, la souche déposée le 3 mars 2009 auprès de la CNCM sous le numéro CNCM I-4132, et la souche déposée le 3 mars 2009 auprès de la CNCM sous le numéro CNCM I-4133.

6. Utilisation d'une souche selon l'une quelconque des revendications 1 à 5 pour la production de levures en présence d'un mélange de glycérol et de sucre, ledit mélange comprenant une proportion de glycérol d'au moins 5% en équivalent saccharose, la somme des proportions de glycérol et de sucre dudit mélange étant égale à 100% en équivalent saccharose.

7. Utilisation d'un mélange de glycérol et de sucre pour la production de levures à partir d'au moins une souche de *Saccharomyces* selon l'une quelconque des revendications 1 à 5, **caractérisée en ce que** ledit mélange comprend une proportion de glycérol d'au moins 5% en équivalent saccharose, la somme des proportions de glycérol et de sucre dudit mélange étant égale à 100% en équivalent saccharose.

8. Procédé de production de levures comprenant les étapes de:
introduction d'une souche de *Saccharomyces* selon l'une quelconque des revendications 1 à 5 dans un fermenteur, et
multiplication de ladite souche en condition aérobie selon un mode semi-continu ou selon un mode continu dans un milieu de culture comprenant un mélange de glycérol et de sucre, ledit mélange comprenant une proportion de glycérol d'au moins 5% en équivalent saccharose, la somme des proportions de glycérol et de sucre dudit mélange étant égale à 100% en équivalent saccharose.

9. Procédé selon la revendication 8, **caractérisée en ce qu'**au moins 80% du glycérol dudit mélange est consommé par ladite souche simultanément à la consommation de sucre, de préférence au moins 85% du glycérol dudit mélange, plus préférentiellement au moins 90% du glycérol dudit mélange, encore plus préférentiellement au moins 95% du glycérol dudit mélange, encore plus préférentiellement au moins 98% du glycérol dudit mélange.

10. Procédé selon l'une quelconque des revendications 8 à 9, **caractérisée en ce que** ledit mélange comprend une proportion de glycérol d'au moins 8% en équivalent saccharose, de préférence au moins 10% en équivalent saccharose, plus préférentiellement au moins 12% en équivalent saccharose, encore plus préférentiellement au moins 15% en équivalent saccharose, encore plus préférentiellement au moins 17% en équivalent saccharose, encore plus préférentiellement au moins 20% en équivalent saccharose.

11. Procédé selon l'une quelconque des revendications 8 à 10, **caractérisé en ce que** ledit mélange comprend une proportion de glycérol d'au moins 30% en équivalent saccharose.

12. Procédé selon l'une quelconque des revendications 8 à 11, **caractérisée en ce que** ledit mélange comprend une proportion de glycérol d'au moins 40% en équivalent saccharose, de préférence au moins 50% en équivalent saccharose, plus préférentiellement au moins 60% en équivalent saccharose, encore plus préférentiellement au moins 70% en équivalent saccharose, encore plus préférentiellement au moins 80% en équivalent saccharose, encore plus préférentiellement au moins 90% en équivalent saccharose.

## Patentansprüche

1. Stamm von *Saccharomyces,* **dadurch gekennzeichnet, dass** er in Gegenwart eines Kulturmediums, welches eine Mischung aus Glycerol und Zucker umfasst, unter aerober Bedingung, mindestens 80% Glycerol der Mischung verbraucht, wenn er gleichzeitig Zucker verbraucht, wobei die Mischung einen Anteil an Glycerol von mindestens 20% Saccharoseäquivalent umfasst, wobei die Summe der Anteile von Glycerol und Zucker der Mischung gleich 100 % Saccharoseäquivalent ist, wobei der Stamm von *Saccharomyces* ein gentechnisch veränderter Stamm ist, um das Gen STL1 in Gegenwart von Zucker zu überexprimieren oder ausgewählt ist aus dem Stamm hinterlegt am 3. März 2009 bei der CNCM unter der Nummer CNCM I-4129, dem Stamm hinterlegt am 3. März 2009 bei der CNCM unter der Nummer CNCM I-4130 und dem Stamm hinterlegt am 3. März 2009 bei der CNCM unter der Nummer CNCM I-4131.

2. Stamm nach Anspruch 1, **dadurch gekennzeichnet, dass** er mindestens 85 % Glycerol der Mischung, vorzugsweise mindestens 90 % Glycerol der Mischung, mehr bevorzugt mindestens 95 % Glycerol der Mischung, noch mehr bevorzugt mindestens 98 % Glycerol der Mischung verbraucht.

3. Stamm nach Anspruch 1 oder Anspruch 2, **dadurch gekennzeichnet, dass** die Mischung einen Anteil an Glycerol von mindestens 30 % Saccharoseäquivalent, vorzugsweise von mindestens 40 % Saccharoseäquivalent, mehr bevorzugt von mindestens 50 % Saccharoseäquivalent, noch mehr bevorzugt von mindestens 60 % Saccharoseäquivalent, noch mehr bevorzugt von mindestens 70 % Saccharoseäquivalent, noch mehr bevorzugt von mindestens 80 % Saccharoseäquivalent, noch mehr bevorzugt von mindestens 90 % Saccharoseäquivalent umfasst.

4. Stamm nach einem der Ansprüche 1 bis 3, **dadurch gekennzeichnet, dass** er ausgewählt aus den Spezies *cerevisiae, boulardii, carlsbergensis* und *uvarum* ist.

5. Stamm nach einem der Ansprüche 1 bis 4, **dadurch gekennzeichnet, dass** er ausgewählt ist aus dem Stamm hinterlegt am 20. Dezember 2007 bei der CNCM unter der Nummer CNCM I-3886, aus dem Stamm hinterlegt am 20. Dezember 2007 bei der CNCM unter der Nummer CNCM I-3887, aus dem Stamm hinterlegt am 20. Dezember 2007 bei der CNCM unter der Nummer CNCM I-3888, aus dem Stamm hinterlegt am 3. Marz 2009 bei der CNCM unter der Nummer CNCM I-4132, und aus dem Stamm hinterlegt am 3. März 2009 bei der CNCM unter der Nummer CNCM I-4133.

6. Verwendung eines Stamms nach einem der Ansprüche 1 bis 5 zur Herstellung von Hefen in Gegenwart einer Mischung aus Glycerol und Zucker, wobei die Mischung einen Anteil an Glycerol von mindestens 5 % Saccharoseäquivalent umfasst, wobei die Summe der Anteile an Glycerol und Zucker der Mischung gleich 100 % Saccharoseäquivalent ist.

7. Verwendung einer Mischung aus Glycerol und Zucker zur Herstellung von Hefen aus mindestens einem Stamm von *Saccharomyces* nach einem der Ansprüche 1 bis 5, **dadurch gekennzeichnet, dass** die Mischung einen Anteil an Glycerol von mindestens 5 % Saccharoseäquivalent umfasst, wobei die Summe der Anteile an Glycerol und Zucker der Mischung gleich 100 % Saccharoseäquivalent ist.

8. Verfahren zur Herstellung von Hefen umfassend die Schritte:
- Einführen eines Stamms von *Saccharomyces* nach einem der Ansprüche 1 bis 5 in einen Fermenter, und
- Vermehren dieses Stammes unter aerober Bedingung in einem halbkontinuierlichen Modus oder in einem kontinuierlichen Modus in einem Kulturmedium umfassend eine Mischung aus Glycerol und Zucker, wobei die Mischung einen Anteil an Glycerol von mindestens 5% Saccharoseäquivalent umfasst, wobei die Summe der Anteile an Glycerol und Zucker der Mischung gleich 100 % Saccharoseäquivalent ist.

9. Verfahren nach Anspruch 8, **dadurch gekennzeichnet, dass** mindestens 80 % Glycerol der Mischung gleichzeitig zu dem Verbrauch von Zucker durch den Stamm verbraucht wird, vorzugsweise mindestens 85 % Glycerol der Mischung, mehr bevorzugt mindestens 90 % Glycerol der Mischung, noch mehr bevorzugt mindestens 95 % Glycerol der Mischung, noch mehr bevorzugt mindestens 98 % Glycerol der Mischung.

10. Verfahren nach einem der Ansprüche 8 bis 9, **dadurch gekennzeichnet, dass** die Mischung einen Anteil an Glycerol von mindestens 8 % Saccharoseäquivalent, vorzugsweise von mindestens 10 % Saccharoseäquivalent, mehr bevorzugt von mindestens 12 % Saccharoseäquivalent, noch mehr bevorzugt von mindestens 15 % Saccharoseäquivalent, noch mehr bevorzugt von mindestens 17 % Saccharoseäquivalent, noch mehr bevorzugt von mindestens 20 % Saccharoseäquivalent umfasst.

11. Verfahren nach einem der Ansprüche 8 bis 10, **dadurch gekennzeichnet, dass** die Mischung einen Anteil an Glycerol von mindestens 30 % Saccharoseäquivalent umfasst.

12. Verfahren nach einem der Ansprüche 8 bis 11, **dadurch gekennzeichnet, dass** die Mischung einen Anteil an Glycerol von mindestens 40 % Saccharoseäquivalent, vorzugsweise von mindestens 50 % Saccharoseäquivalent, mehr bevorzugt von mindestens 60 % Saccharoseäquivalent, noch mehr bevorzugt von mindestens 70 % Saccharoseäquivalent, noch mehr bevorzugt von mindestens 80 % Saccharoseäquivalent, noch mehr bevorzugt von mindestens 90 % Saccharoseäquivalent umfasst.

## Claims

1. *Saccharomyces* Strain, **characterized in that**, in the presence of a culture medium comprising a mixture of glycerol and sugar, in aerobic conditions, it consumes at least 80% of the glycerol of said mixture while simultaneously consuming sugar, said mixture comprising a proportion of glycerol of at least 20% sucrose equivalent, the sum of the proportions of glycerol and of sugar of said mixture being equal to 100% sucrose equivalent, the *Saccharomyces* strain being a strain genetically modified to overexpress the STL1 gene in the presence of sugar or being chosen from the strain deposited on 3 March 2009 with the CNCM under number CNCM I-4129, the strain deposited on 3 March 2009 with the CNCM under number CNCM I-4130 and the strain deposited on 3 March 2009 with the CNCM under number CNCM I-4131.

2. Strain according to claim 1, **characterized in that** it consumes at least 85% of the glycerol of the mixture, preferably at least 90% of the glycerol of the mixture, more preferably at least 95% of the glycerol of the mixture and even more preferably at least 98% of the glycerol of the mixture.

3. Strain according to claim 1 or claim 2, **characterized in that** said mixture comprises a proportion of glycerol of at least 30% sucrose equivalent, preferably at least 40% sucrose equivalent, more preferably at least 50% sucrose equivalent, even more preferably at least 60% sucrose equivalent, even more preferably at least 70% sucrose equivalent, even more preferably at least 80% sucrose equivalent, even more preferably at least 90% sucrose equivalent.

4. Strain according to one of Claims 1 to 3, **characterized in that** it is chosen from the species *cerevisiae, boulardii*, *carlsbergensis* and *uvarum.*

5. Strain according to any one of claims 1 to 4, **characterized in that** it is chosen from the strain deposited on 20 December 2007 with the CNCM under number CNCM I-3886, the strain deposited on 20 December 2007 with the CNCM under number CNCM I-3887, the strain deposited on 20 December 2007 with the CNCM under number CNCM I-3888, the strain deposited on 3 March 2009 with the CNCM under number CNCM I-4132, and the strain deposited on 3 March 2009 with the CNCM under number CNCM I-4133.

6. Use of a strain according to any one of claims 1 to 5 for producing yeasts in the presence of a mixture of glycerol and sugar, said mixture comprising a proportion of glycerol of at least 5% sucrose equivalent, the sum of the proportions of glycerol and of sugar of said mixture being equal to 100% sucrose equivalent.

7. Use of a mixture of glycerol and of sugar for producing yeasts from at least one *Saccharomyces* strain according to any one of claims 1 to 5, **characterized in that** said mixture comprises a proportion of glycerol of at least 5% sucrose equivalent, the sum of the proportions of glycerol and of sugar of said mixture being equal to 100% sucrose equivalent.

8. Process for producing yeasts, comprising the steps of:
introducing a strain of *Saccharomyces* according to any one of Claims 1 to 5 into a fermenter, and
multiplying said strain in aerobic conditions in a semi-continuous mode or in a continuous mode, in a culture medium comprising a mixture of glycerol and sugar, said mixture comprising a proportion of glycerol of at least 5% sucrose equivalent, the sum of the proportions of glycerol and of sugar of said mixture being equal to 100% sucrose equivalent.

9. Process according to claim 8, **characterized in that** at least 80% of the glycerol of said mixture is consumed by said strain simultaneously with the consumption of sugar, preferably at least 85% of the glycerol of said mixture, more preferably at least 90% of the glycerol of said mixture, even more preferably at least 95% of the glycerol of said mixture, even more preferably at least 98% of the glycerol of said mixture.

10. Process according to any one of claims 8 and 9, **characterized in that** said mixture comprises a proportion of glycerol of at least 8% sucrose equivalent, preferably at least 10% sucrose equivalent, more preferably at least 12% sucrose equivalent, even more preferably at least 15% sucrose equivalent, even more preferably at least 17% sucrose equivalent, even more preferably at least 20% sucrose equivalent.

11. Process according to any one of claims 8 to 10, **characterized in that** said mixture comprises a proportion of glycerol of at least 30% sucrose equivalent.

12. Process according to any one of claims 8 to 11, **characterized in that** said mixture comprises a proportion of glycerol of at least 40% sucrose equivalent, preferably at least 50% sucrose equivalent, more preferably at least 60% sucrose equivalent, even more preferably at least 70% sucrose equivalent, even more preferably at least 80% sucrose equivalent, even more preferably at least 90% sucrose equivalent.
